# EUROPEAN PATENT SPECIFICATION

(11) **EP 3 940 081 B1**
(45) Date of publication and mention of the grant of the patent: **11.06.2025**
(21) Application number: 20794443.0
(22) Date of filing: 13.04.2020
(51) Int. Cl.: C12Q 1/04, C12Q 1/37, G01N 33/48, G01N 21/77, G01N 21/78, G01N 33/532, G01N 33/569

(54) **PERIODONTOPATHIC BACTERIA DETECTION METHOD**
VERFAHREN ZUR DETEKTION VON PERIODONTOPATHISCHEN BAKTERIEN
PROCÉDÉ DE DÉTECTION DE BACTÉRIES PARODONTOPATHIQUES

(30) Priority: 25.04.2019 JP 2019083482
(43) Date of publication of application: 19.01.2022
(73) Proprietor: Adtec Co., Ltd., Usa-shi, Oita 879-0453 (JP)
(72) Inventor: YAMASHITA , Yuri, Usa-shi, Oita 879-0453 (JP); ITAGAKI , Hatsue, Usa-shi, Oita 879-0453 (JP); MORIWAKA, Senta, Usa-shi, Oita 879-0453 (JP); KOBAYASHI, Kaoru, Usa-shi, Oita 879-0453 (JP); KOBAYASHI , Yukuharu, Usa-shi, Oita 879-0453 (JP)
(74) Representative: Algemeen Octrooi- en Merkenbureau B.V.
(86) International application number: PCT/JP2020/016256
(87) International publication number: WO 2020/218052

(56) References cited:
- WO-A1-2017/090296
- JP-A- 2017 093 358

## Description

### FIELD OF THE ART

The present invention relates to a method for detecting periodontopathic bacteria, and more specifically to a method for detecting periodontopathic bacteria which does not require special equipment, allowing periodontopathic bacteria that are measured under high temperature conditions, to be detected at room temperature in less than 10 minutes (about 2 to 8 minutes).

### BACKGROUND ART

It is said that approximately 80% of adults in Japan are affected by periodontal disease. Periodontal disease causes tooth loss and it has become clear that, in accompaniment, not only are dysgeusia and abnormal salivation caused but abnormalities of the central nervous system and the autonomic nervous system are also caused. Further in recent years, it has come to be noted that periodontal disease is a risk factor of various systemic disorders, such as coronary heart disease, cerebral infarction, etc.

Methods for inspecting bacterial infection and inflammation, among inspection items of periodontal disease, include a method of visually judging stained tooth surfaces using a plaque staining solution, plaque adhesion condition inspection, with which staining is not performed and tooth surfaces are abraded with a tip of dental probe or dental explorer, etc., to judge whether or not plaque is adhered, periodontopathic bacteria inspection, with which subgingival plaque is sampled with a paper point and an inspection agency is requested to measure bacterial count by a DNA quantitation method, etc., an antibody inspection method, with which an IgG antibody titer in serum is measured for periodontopathic bacteria, etc. These methods not only require time, labor, and cost but also require special facilities and skills and therefore do not enable a plurality of patients to be inspected at the same time in a simple and rapid manner and the burden on a patient is also large.

Among periodontopathic bacteria, the three types of bacteria, Porphyromonas gingivalis (P. g), Treponema denticola (T. d), and Tannerella forsythia (T. f) are referred to as the red complex, which is considered to be the most dangerous bacterial types group that causes severe periodontal disease. These three types of bacteria have arginine-specific peptidase activity (trypsin-like enzymatic activity) and methods of detecting trypsin-like enzymatic activity using a synthetic substrate are reported in Patent Documents 1 to 4.

Also, it is known that this enzyme activity is activated by a reducing agent (Patent Documents 3 and 4, Non-Patent Document 1). It has also been reported that the enzyme activity can be analyzed at room temperature in 10 minutes. (Patent Document 4).

If the trypsin-like enzymatic activity can be analyzed and judged in a shorter time and a simpler manner for the presence of the three important types of bacteria among periodontopathic bacteria, this would be a useful method for screening for periodontal disease.

### PRIOR ART DOCUMENTS

### PATENT DOCUMENTS

Patent Document 1: Japanese Patent Application Publication (Translation of PCT Application) No. H08-500241
Patent Document 2: Japanese Unexamined Patent Application Publication No. H05-317095
Patent Document 3: International Publication No. WO 2004/106541
Patent Document 4: Japanese Unexamined Patent Application Publication No. 2017-93358

Non-Patent Document 1: Wendy E. Kaman et al. "Highly Specific Protease-Based Approach for Detection of Porphyromonus gingivalis in Diagnosis of periodontitis," Journal of Clinical Microbiology, vol. 50, Number 1, pp. 104-112, 2012.
WO2017/090296 discloses a method for analyzing the presence of periodontal disease-causing bacteria by analyzing the trypsin-like protease activity specific to periodontal disease-causing bacteria using a first compound having an antioxidant action and / or a second compound having an action of protecting the SH group and cleaving a disulfide bond. The analysis required 10 minutes at room temperature.

### SUMMARY OF THE INVENTION

### PROBLEM TO BE SOLVED BY THE INVENTION

However, with trypsin-like enzymatic activity, sufficient sensitivity cannot be obtained other than at its optimal temperature of 50 to 60°C and for measurement, special equipment capable of maintaining the optimal temperature is necessary and this is an obstacle to dissemination of inspection of periodontopathic bacteria. Analyzing enzyme activity after a period of 10 minutes at room temperature also lengthens the time constraint for the patient. It has therefore been necessary to immediately detect periodontopathic bacteria at locations where samples are sampled from patients.

The present inventors, focusing on the chemical properties of P.g, T.d and T.f, have found that if a compound having an antioxidant effect or a compound having the action of protecting SH groups (mercapto groups) and cleaving disulfide bonds is present when analyzing trypsin-like enzymatic activity, then it is possible to analyze trypsin-like enzymatic activity with high sensitivity at room temperature in less than 10 minutes, and have thereupon completed the present invention.

It is an object of the present invention to provide a method of analyzing periodontopathic bacteria that does not require special equipment, and that allows trypsin-like enzymatic activity specific to three types of periodontopathic bacteria, P.g, T.d and T.f, which are measured under high temperature conditions, to be analyzed with high sensitivity at room temperature in less than 10 minutes (about 2 to 8 minutes).

### MEANS FOR SOLVING THE PROBLEM

One aspect of the invention is defined in claim
1. One aspect of the invention is defined in claim 5

One aspect of the invention is defined in claim 2.

One aspect of the invention is defined in claim 3.

One aspect of the invention is defined in claim 4.

The first compound is a chemical substance having an antioxidant effect, and it is selected from among L-ascorbic acid, L-cysteine hydrochloride, and glutathione.

The second compound is a chemical substance having action of protecting SH groups and cleaving disulfide bonds, and it is selected from among DTT (dithiothreitol), thioglycolic acid, thioglycerol, and mercaptoethanol.

It will be appreciated that the second compound cannot be DTT for the methods of claims 3 and 4.

In the analysis of the trypsin-like protease activity, one of either of the first compound and the second compound may be present or both the first compound and the second compound may be mixed.

Room temperature indicates a state of not being heated or cooled from an external system. It is approximately 20°C to 30°C.

The sample may be any sample to be analyzed such as an oral cavity swab sample, plaque sample, tongue swab or tongue coating sample or saliva, or a swab from an implant, bridge or denture removed from the mouth, in the form of a solution diluted with an appropriate medium or an extract liquid obtained by extraction of periodontopathic bacteria from the sample to be analyzed using an appropriate medium.

The first compound and the second compound may be incorporated, together with the substrate, in a water absorbent material (dry retention method). The substrate used is N-α-benzoyl-DL-arginine-2-naphthylamide hydrochloride, a substance that causes chemical reaction when acted upon by an enzyme of a periodontopathic bacterium contained in a sample. The reagent is incorporated in the water absorbent material (test specimen or carrier), the analysis object sample and the water absorbent material are put in direct contact, and presence or non-presence of a coloring substance that is released by enzyme activity of the periodontopathic bacteria is analyzed. In this case, a coloring reagent may be used as necessary.

As the coloring reagent, any of various known reagents may be used. For example, sodium 4-hydroxy-3-[(2,4-dihydroxy-3-quinolyl)azo]benzenesulfonate, 4-benzoylamino-2,5-diethoxybenzenediazonium, 4-(dimethylamino)cinnamaldehyde, etc., may be used.

The water absorbent material is not restricted in particular as long as it is a carrier having a water absorbing property and capable of incorporating the first compound and/or the second compound. Paper, filter paper, cellulose, nonwoven fabric, glass fiber, porous filter, or cotton, etc., can be cited as examples. The substrate may also be incorporated in the water absorbent material. The water absorbent material may be used as it is or may be provided on a suitable member, for example, waterproof paper, glass, plastic, wood, or metal, etc., to facilitate handling. Shape, length, and thickness of the water absorbent material are not restricted in particular as long as the sample can be contacted.

As the dry retention method, the first compound and the second compound may be dissolved or dispersed in a suitable solvent together with or separately of the substrate and the solution (or dispersion) may be incorporated in the water absorbent material. As the solvent, for example, tris-hydrochloride buffer solution, tris-maleate buffer solution, phosphate buffer solution, glycine buffer solution, sodium borate buffer solution, carbonate-bicarbonate buffer solution, Good's buffer solution, or purified water, etc., may be used. Further, an abovementioned solvent added with a suitable surfactant, for example, polyoxyethylene sorbitan monolaurate, polyoxyethylene sorbitan monooleate, octylphenol ethoxylate, etc., may be used.

The water absorbent material may be impregnated with 10 µL to 500 µL of a 1 mM to 100 mM solution (dispersion) of the first compound and/or the second compound and thereafter used upon drying by air drying, blow drying, depressurized vacuum drying, or freeze drying.

The first compound and the second compound may be dissolved in a suitable solvent and thereafter added to the sample (solution method). Also, the first compound and the second compound may be added directly to the sample.

As the solvent, for example, tris-hydrochloride buffer solution, tris-maleate buffer solution, phosphate buffer solution, glycine buffer solution, sodium borate buffer solution, carbonate-bicarbonate buffer solution, Good's buffer solution, or purified water, etc., may be used. Further, an abovementioned solvent added with a suitable surfactant, for example, polyoxyethylene sorbitan monolaurate, polyoxyethylene sorbitan monooleate, octylphenol ethoxylate, etc., may be used.

A 10 mM to 1000 mM solution of the first compound and the second compound of 1/10 volume of the volume of the sample may be added to the sample. Also, if the first compound and the second compound are to be added directly to the sample, the addition amount is approximately 1 mM to 100 mM.

The invention according to claim 5 is the method for detecting periodontopathic bacteria according to any one of claims 1 to 4, wherein the concentration of the first compound is 6.25 mM to 100 mM.

The invention according to claim 6 is the method for detecting periodontopathic bacteria according to any one of claims 1 to 5, wherein the concentration of the second compound is 6.25 mM to 100 mM.

If the concentration of the first compound is in the range of 6.25 mM to 100 mM, the second compound is DTT, thioglycolic acid, thioglycerol, or mercaptoethanol and its concentration is in the range of 6.25 mM to 100 mM, it will be possible to detect a periodontopathic bacterium in about 2 minutes. It is therefore possible to analyze and judge the presence of periodontopathic bacteria in a very short time, which is highly effective for screening of periodontal disease.

### EFFECT(S) OF THE INVENTION

According to the present invention, a first compound having an antioxidant effect and a second compound having action of protecting SH groups and cleaving disulfide bonds is added to a sample, whereby trypsin-like enzymatic activity specific to the three types of bacteria P.g, T.d and T.f, which has conventionally been measured under high temperature conditions, can be analyzed at room temperature in less than 10 minutes. This allows convenient analysis and determination of trypsin-like enzymatic activity in a short period of time, which is effective for screening of periodontal disease.

### BRIEF DESCRIPTION OF THE DRAWINGS

Fig. 1 is a graph showing detection sensitivities in the absence of a first compound and second compound (Comparative Example 1), under the same conditions as the analysis method for periodontopathic bacteria according to Example 1 and Example 3 of the invention.
Fig. 2 is a graph showing P.g detection sensitivities at different analysis times, in a method of analyzing periodontopathic bacteria using a dry retention method with 6.25 mM L-ascorbic acid (Example 1).
Fig. 3 is a graph showing P.g detection sensitivities at different analysis times, in a method of analyzing periodontopathic bacteria using a dry retention method with 12.5 mM L-ascorbic acid (Example 1).
Fig. 4 is a graph showing P.g detection sensitivities at different analysis times, in a method of analyzing periodontopathic bacteria using a dry retention method with 25 mM L-ascorbic acid (Example 1).
Fig. 5 is a graph showing P.g detection sensitivities at different analysis times, in a method of analyzing periodontopathic bacteria using a dry retention method with 50 mM L-ascorbic acid (Example 1).
Fig. 6 is a graph showing P.g detection sensitivities at different analysis times, in a method of analyzing periodontopathic bacteria using a dry retention method with 100 mM L-ascorbic acid (Example 1).
Fig. 7 is a graph showing P.g detection sensitivities at different analysis times, in a method of analyzing periodontopathic bacteria using a dry retention method with 6.25 mM L-cysteine hydrochloride (Example 1).
Fig. 8 is a graph showing P.g detection sensitivities at different analysis times, in a method of analyzing periodontopathic bacteria using a dry retention method with 12.5 mM L-cysteine hydrochloride (Example 1).
Fig. 9 is a graph showing P.g detection sensitivities at different analysis times, in a method of analyzing periodontopathic bacteria using a dry retention method with 25 mM L-cysteine hydrochloride (Example 1).
Fig. 10 is a graph showing P.g detection sensitivities at different analysis times, in a method of analyzing periodontopathic bacteria using a dry retention method with 50 mM L-cysteine hydrochloride (Example 1).
Fig. 11 is a graph showing P.g detection sensitivities at different analysis times, in a method of analyzing periodontopathic bacteria using a dry retention method with 100 mM L-cysteine hydrochloride (Example 1).
Fig. 12 is a graph showing P.g detection sensitivities at different analysis times, in a method of analyzing periodontopathic bacteria using a dry retention method with 6.25 mM glutathione (Example 1).
Fig. 13 is a graph showing P.g detection sensitivities at different analysis times, in a method of analyzing periodontopathic bacteria using a dry retention method with 12.5 mM glutathione (Example 1).
Fig. 14 is a graph showing P.g detection sensitivities at different analysis times, in a method of analyzing periodontopathic bacteria using a dry retention method with 25 mM glutathione (Example 1).
Fig. 15 is a graph showing P.g detection sensitivities at different analysis times, in a method of analyzing periodontopathic bacteria using a dry retention method with 50 mM glutathione (Example 1).
Fig. 16 is a graph showing P.g detection sensitivities at different analysis times, in a method of analyzing periodontopathic bacteria using a dry retention method with 100 mM glutathione (Example 1).
Fig. 17 is a graph showing P.g detection sensitivities at different analysis times, in a method of analyzing periodontopathic bacteria using a dry retention method with 6.25 mM DTT (dithiothreitol) (Example 1).
Fig. 18 is a graph showing P.g detection sensitivities at different analysis times, in a method of analyzing periodontopathic bacteria using a dry retention method with 12.5 mM DTT (Example 1).
Fig. 19 is a graph showing P.g detection sensitivities at different analysis times, in a method of analyzing periodontopathic bacteria using a dry retention method with 25 mM DTT (Example 1).
Fig. 20 is a graph showing P.g detection sensitivities at different analysis times, in a method of analyzing periodontopathic bacteria using a dry retention method with 50 mM DTT (Example 1).
Fig. 21 is a graph showing P.g detection sensitivities at different analysis times, in a method of analyzing periodontopathic bacteria using a dry retention method with 100 mM DTT (Example 1).
Fig. 22 is a graph showing P.g detection sensitivities at different analysis times, in a method of analyzing periodontopathic bacteria using a dry retention method with 6.25 mM thioglycolic acid (Example 1).
Fig. 23 is a graph showing P.g detection sensitivities at different analysis times, in a method of analyzing periodontopathic bacteria using a dry retention method with 12.5 mM thioglycolic acid (Example 1).
Fig. 24 is a graph showing P.g detection sensitivities at different analysis times, in a method of analyzing periodontopathic bacteria using a dry retention method with 25 mM thioglycolic acid (Example 1).
Fig. 25 is a graph showing P.g detection sensitivities at different analysis times, in a method of analyzing periodontopathic bacteria using a dry retention method with 50 mM thioglycolic acid (Example 1).
Fig. 26 is a graph showing P.g detection sensitivities at different analysis times, in a method of analyzing periodontopathic bacteria using a dry retention method with 100 mM thioglycolic acid (Example 1).
Fig. 27 is a graph showing P.g detection sensitivities at different analysis times, in a method of analyzing periodontopathic bacteria using a dry retention method with 6.25 mM thioglycerol (Example 1).
Fig. 28 is a graph showing P.g detection sensitivities at different analysis times, in a method of analyzing periodontopathic bacteria using a dry retention method with 12.5 mM thioglycerol (Example 1).
Fig. 29 is a graph showing P.g detection sensitivities at different analysis times, in a method of analyzing periodontopathic bacteria using a dry retention method with 25 mM thioglycerol (Example 1).
Fig. 30 is a graph showing P.g detection sensitivities at different analysis times, in a method of analyzing periodontopathic bacteria using a dry retention method with 50 mM thioglycerol (Example 1).
Fig. 31 is a graph showing P.g detection sensitivities at different analysis times, in a method of analyzing periodontopathic bacteria using a dry retention method with 100 mM thioglycerol (Example 1).
Fig. 32 is a graph showing P.g detection sensitivities at different analysis times, in a method of analyzing periodontopathic bacteria using a dry retention method with 6.25 mM mercaptoethanol (Example 1).
Fig. 33 is a graph showing P.g detection sensitivities at different analysis times, in a method of analyzing periodontopathic bacteria using a dry retention method with 12.5 mM mercaptoethanol (Example 1).
Fig. 34 is a graph showing P.g detection sensitivities at different analysis times, in a method of analyzing periodontopathic bacteria using a dry retention method with 25 mM mercaptoethanol (Example 1).
Fig. 35 is a graph showing P.g detection sensitivities at different analysis times, in a method of analyzing periodontopathic bacteria using a dry retention method with 50 mM mercaptoethanol (Example 1).
Fig. 36 is a graph showing P.g detection sensitivities at different analysis times, in a method of analyzing periodontopathic bacteria using a dry retention method with 100 mM mercaptoethanol (Example 1).
Fig. 37 is a graph showing P.g detection sensitivities at different analysis times, in a method of analyzing periodontopathic bacteria using a dry retention method with 6.25 mM TCEP (tris(2-carboxyethyl)phosphine hydrochloride) (Example 1).
Fig. 38 is a graph showing P.g detection sensitivities at different analysis times, in a method of analyzing periodontopathic bacteria using a dry retention method with 12.5 mM TCEP (Example 1).
Fig. 39 is a graph showing P.g detection sensitivities at different analysis times, in a method of analyzing periodontopathic bacteria using a dry retention method with 25 mM TCEP (Example 1).
Fig. 40 is a graph showing P.g detection sensitivities at different analysis times, in a method of analyzing periodontopathic bacteria using a dry retention method with 50 mM TCEP (Example 1).
Fig. 41 is a graph showing P.g detection sensitivities at different analysis times, in a method of analyzing periodontopathic bacteria using a dry retention method with 100 mM TCEP (Example 1).
Fig. 42 is a graph showing detection sensitivities in the absence of the first compound and second compound (Comparative Example 2), under the same conditions as the analysis method for periodontopathic bacteria according to Example 2 and Example 4 of the invention.
Fig. 43 is a graph showing P.g detection sensitivities at different analysis times, in a method of analyzing periodontopathic bacteria using a solution method with 6.25 mM L-ascorbic acid (Example 2).
Fig. 44 is a graph showing P.g detection sensitivities at different analysis times, in a method of analyzing periodontopathic bacteria using a solution method with 12.5 mM L-ascorbic acid (Example 2).
Fig. 45 is a graph showing P.g detection sensitivities at different analysis times, in a method of analyzing periodontopathic bacteria using a solution method with 25 mM L-ascorbic acid (Example 2).
Fig. 46 is a graph showing P.g detection sensitivities at different analysis times, in a method of analyzing periodontopathic bacteria using a solution method with 50 mM L-ascorbic acid (Example 2).
Fig. 47 is a graph showing P.g detection sensitivities at different analysis times, in a method of analyzing periodontopathic bacteria using a solution method with 100 mM L-ascorbic acid (Example 2).
Fig. 48 is a graph showing P.g detection sensitivities at different analysis times, in a method of analyzing periodontopathic bacteria using a solution method with 6.25 mM L-cysteine hydrochloride (Example 2).
Fig. 49 is a graph showing P.g detection sensitivities at different analysis times, in a method of analyzing periodontopathic bacteria using a solution method with 12.5 mM L-cysteine hydrochloride (Example 2).
Fig. 50 is a graph showing P.g detection sensitivities at different analysis times, in a method of analyzing periodontopathic bacteria using a solution method with 25 mM L-cysteine hydrochloride (Example 2).
Fig. 51 is a graph showing P.g detection sensitivities at different analysis times, in a method of analyzing periodontopathic bacteria using a solution method with 50 mM L-cysteine hydrochloride (Example 2).
Fig. 52 is a graph showing P.g detection sensitivities at different analysis times, in a method of analyzing periodontopathic bacteria using a solution method with 100 mM L-cysteine hydrochloride (Example 2).
Fig. 53 is a graph showing P.g detection sensitivities at different analysis times, in a method of analyzing periodontopathic bacteria using a solution method with 6.25 mM glutathione (Example 2).
Fig. 54 is a graph showing P.g detection sensitivities at different analysis times, in a method of analyzing periodontopathic bacteria using a solution method with 12.5 mM glutathione (Example 2).
Fig. 55 is a graph showing P.g detection sensitivities at different analysis times, in a method of analyzing periodontopathic bacteria using a solution method with 25 mM glutathione (Example 2).
Fig. 56 is a graph showing P.g detection sensitivities at different analysis times, in a method of analyzing periodontopathic bacteria using a solution method with 50 mM glutathione (Example 2).
Fig. 57 is a graph showing P.g detection sensitivities at different analysis times, in a method of analyzing periodontopathic bacteria using a solution method with 100 mM glutathione (Example 2).
Fig. 58 is a graph showing P.g detection sensitivities at different analysis times, in a method of analyzing periodontopathic bacteria using a solution method with 6.25 mM DTT (Example 2).
Fig. 59 is a graph showing P.g detection sensitivities at different analysis times, in a method of analyzing periodontopathic bacteria using a solution method with 12.5 mM DTT (Example 2).
Fig. 60 is a graph showing P.g detection sensitivities at different analysis times, in a method of analyzing periodontopathic bacteria using a solution method with 25 mM DTT (Example 2).
Fig. 61 is a graph showing P.g detection sensitivities at different analysis times, in a method of analyzing periodontopathic bacteria using a solution method with 50 mM DTT (Example 2).
Fig. 62 is a graph showing P.g detection sensitivities at different analysis times, in a method of analyzing periodontopathic bacteria using a solution method with 100 mM DTT (Example 2).
Fig. 63 is a graph showing P.g detection sensitivities at different analysis times, in a method of analyzing periodontopathic bacteria using a solution method with 6.25 mM thioglycolic acid (Example 2).
Fig. 64 is a graph showing P.g detection sensitivities at different analysis times, in a method of analyzing periodontopathic bacteria using a solution method with 12.5 mM thioglycolic acid (Example 2).
Fig. 65 is a graph showing P.g detection sensitivities at different analysis times, in a method of analyzing periodontopathic bacteria using a solution method with 25 mM thioglycolic acid (Example 2).
Fig. 66 is a graph showing P.g detection sensitivities at different analysis times, in a method of analyzing periodontopathic bacteria using a solution method with 50 mM thioglycolic acid (Example 2).
Fig. 67 is a graph showing P.g detection sensitivities at different analysis times, in a method of analyzing periodontopathic bacteria using a solution method with 100 mM thioglycolic acid (Example 2).
Fig. 68 is a graph showing P.g detection sensitivities at different analysis times, in a method of analyzing periodontopathic bacteria using a solution method with 6.25 mM thioglycerol (Example 2).
Fig. 69 is a graph showing P.g detection sensitivities at different analysis times, in a method of analyzing periodontopathic bacteria using a solution method with 12.5 mM thioglycerol (Example 2).
Fig. 70 is a graph showing P.g detection sensitivities at different analysis times, in a method of analyzing periodontopathic bacteria using a solution method with 25 mM thioglycerol (Example 2).
Fig. 71 is a graph showing P.g detection sensitivities at different analysis times, in a method of analyzing periodontopathic bacteria using a solution method with 50 mM thioglycerol (Example 2).
Fig. 72 is a graph showing P.g detection sensitivities at different analysis times, in a method of analyzing periodontopathic bacteria using a solution method with 100 mM thioglycerol (Example 2).
Fig. 73 is a graph showing P.g detection sensitivities at different analysis times, in a method of analyzing periodontopathic bacteria using a solution method with 6.25 mM mercaptoethanol (Example 2).
Fig. 74 is a graph showing P.g detection sensitivities at different analysis times, in a method of analyzing periodontopathic bacteria using a solution method with 12.5 mM mercaptoethanol (Example 2).
Fig. 75 is a graph showing P.g detection sensitivities at different analysis times, in a method of analyzing periodontopathic bacteria using a solution method with 25 mM mercaptoethanol (Example 2).
Fig. 76 is a graph showing P.g detection sensitivities at different analysis times, in a method of analyzing periodontopathic bacteria using a solution method with 50 mM mercaptoethanol (Example 2).
Fig. 77 is a graph showing P.g detection sensitivities at different analysis times, in a method of analyzing periodontopathic bacteria using a solution method with 100 mM mercaptoethanol (Example 2).
Fig. 78 is a graph showing P.g detection sensitivities at different analysis times, in a method of analyzing periodontopathic bacteria using a solution method with 6.25 mM TCEP (Example 2).
Fig. 79 is a graph showing P.g detection sensitivities at different analysis times, in a method of analyzing periodontopathic bacteria using a solution method with 12.5 mM TCEP (Example 2).
Fig. 80 is a graph showing P.g detection sensitivities at different analysis times, in a method of analyzing periodontopathic bacteria using a solution method with 25 mM TCEP (Example 2).
Fig. 81 is a graph showing P.g detection sensitivities at different analysis times, in a method of analyzing periodontopathic bacteria using a solution method with 50 mM TCEP (Example 2).
Fig. 82 is a graph showing P.g detection sensitivities at different analysis times, in a method of analyzing periodontopathic bacteria using a solution method with 100 mM TCEP (Example 2).
Fig. 83 is a graph showing P.g detection sensitivities at different analysis times, in a method of analyzing periodontopathic bacteria using a dry retention method with a mixture of 25 mM L-ascorbic acid and 25 mM DTT (Example 3).
Fig. 84 is a graph showing P.g detection sensitivities at different analysis times, in a method of analyzing periodontopathic bacteria using a dry retention method with a mixture of 25 mM L-ascorbic acid and 25 mM thioglycolic acid (Example 3).
Fig. 85 is a graph showing P.g detection sensitivities at different analysis times, in a method of analyzing periodontopathic bacteria using a dry retention method with a mixture of 25 mM L-ascorbic acid and 25 mM thioglycerol (Example 3).
Fig. 86 is a graph showing P.g detection sensitivities at different analysis times, in a method of analyzing periodontopathic bacteria using a dry retention method with a mixture of 25 mM L-ascorbic acid and 25 mM mercaptoethanol (Example 3).
Fig. 87 is a graph showing P.g detection sensitivities at different analysis times, in a method of analyzing periodontopathic bacteria using a dry retention method with a mixture of 25 mM L-ascorbic acid and 25 mM TCEP (Example 3).
Fig. 88 is a graph showing P.g detection sensitivities at different analysis times, in a method of analyzing periodontopathic bacteria using a dry retention method with a mixture of 25 mM L-cysteine hydrochloride and 25 mM DTT (Example 3).
Fig. 89 is a graph showing P.g detection sensitivities at different analysis times, in a method of analyzing periodontopathic bacteria using a dry retention method with a mixture of 25 mM L-cysteine hydrochloride and 25 mM thioglycolic acid (Example 3).
Fig. 90 is a graph showing P.g detection sensitivities at different analysis times, in a method of analyzing periodontopathic bacteria using a dry retention method with a mixture of 25 mM L-cysteine hydrochloride and 25 mM thioglycerol (Example 3).
Fig. 91 is a graph showing P.g detection sensitivities at different analysis times, in a method of analyzing periodontopathic bacteria using a dry retention method with a mixture of 25 mM L-cysteine hydrochloride and 25 mM mercaptoethanol (Example 3).
Fig. 92 is a graph showing P.g detection sensitivities at different analysis times, in a method of analyzing periodontopathic bacteria using a dry retention method with a mixture of 25 mM L-cysteine hydrochloride and 25 mM TCEP (Example 3).
Fig. 93 is a graph showing P.g detection sensitivities at different analysis times, in a method of analyzing periodontopathic bacteria using a dry retention method with a mixture of 25 mM glutathione and 25 mM DTT (Example 3).
Fig. 94 is a graph showing P.g detection sensitivities at different analysis times, in a method of analyzing periodontopathic bacteria using a dry retention method with a mixture of 25 mM glutathione and 25 mM thioglycolic acid (Example 3).
Fig. 95 is a graph showing P.g detection sensitivities at different analysis times, in a method of analyzing periodontopathic bacteria using a dry retention method with a mixture of 25 mM glutathione and 25 mM thioglycerol (Example 3).
Fig. 96 is a graph showing P.g detection sensitivities at different analysis times, in a method of analyzing periodontopathic bacteria using a dry retention method with a mixture of 25 mM glutathione and 25 mM mercaptoethanol (Example 3).
Fig. 97 is a graph showing P.g detection sensitivities at different analysis times, in a method of analyzing periodontopathic bacteria using a dry retention method with a mixture of 25 mM glutathione and 25 mM TCEP (Example 3).
Fig. 98 is a graph showing P.g detection sensitivities at different analysis times, in a method of analyzing periodontopathic bacteria using a solution method with a mixture of 25 mM L-ascorbic acid and 25 mM DTT (Example 4).
Fig. 99 is a graph showing P.g detection sensitivities at different analysis times, in a method of analyzing periodontopathic bacteria using a solution method with a mixture of 25 mM L-ascorbic acid and 25 mM thioglycolic acid (Example 4).
Fig. 100 is a graph showing P.g detection sensitivities at different analysis times, in a method of analyzing periodontopathic bacteria using a solution method with a mixture of 25 mM L-ascorbic acid and 25 mM thioglycerol (Example 4).
Fig. 101 is a graph showing P.g detection sensitivities at different analysis times, in a method of analyzing periodontopathic bacteria using a solution method with a mixture of 25 mM L-ascorbic acid and 25 mM mercaptoethanol (Example 4).
Fig. 102 is a graph showing P.g detection sensitivities at different analysis times, in a method of analyzing periodontopathic bacteria using a solution method with a mixture of 25 mM L-ascorbic acid and 25 mM TCEP (Example 4).
Fig. 103 is a graph showing P.g detection sensitivities at different analysis times, in a method of analyzing periodontopathic bacteria using a solution method with a mixture of 25 mM L-cysteine hydrochloride and 25 mM DTT (Example 4).
Fig. 104 is a graph showing P.g detection sensitivities at different analysis times, in a method of analyzing periodontopathic bacteria using a solution method with a mixture of 25 mM L-cysteine hydrochloride and 25 mM thioglycolic acid (Example 4).
Fig. 105 is a graph showing P.g detection sensitivities at different analysis times, in a method of analyzing periodontopathic bacteria using a solution method with a mixture of 25 mM L-cysteine hydrochloride and 25 mM thioglycerol (Example 4).
Fig. 106 is a graph showing P.g detection sensitivities at different analysis times, in a method of analyzing periodontopathic bacteria using a solution method with a mixture of 25 mM L-cysteine hydrochloride and 25 mM mercaptoethanol (Example 4).
Fig. 107 is a graph showing P.g detection sensitivities at different analysis times, in a method of analyzing periodontopathic bacteria using a solution method with a mixture of 25 mM L-cysteine hydrochloride and 25 mM TCEP (Example 4).
Fig. 108 is a graph showing P.g detection sensitivities at different analysis times, in a method of analyzing periodontopathic bacteria using a solution method with a mixture of 25 mM glutathione and 25 mM DTT (Example 4).
Fig. 109 is a graph showing P.g detection sensitivities at different analysis times, in a method of analyzing periodontopathic bacteria using a solution method with a mixture of 25 mM glutathione and 25 mM thioglycolic acid (Example 4).
Fig. 110 is a graph showing P.g detection sensitivities at different analysis times, in a method of analyzing periodontopathic bacteria using a solution method with a mixture of 25 mM glutathione and 25 mM thioglycerol (Example 4).
Fig. 111 is a graph showing P.g detection sensitivities at different analysis times, in a method of analyzing periodontopathic bacteria using a solution method with a mixture of 25 mM glutathione and 25 mM mercaptoethanol (Example 4).
Fig. 112 is a graph showing P.g detection sensitivities at different analysis times, in a method of analyzing periodontopathic bacteria using a solution method with a mixture of 25 mM glutathione and 25 mM TCEP (Example 4).

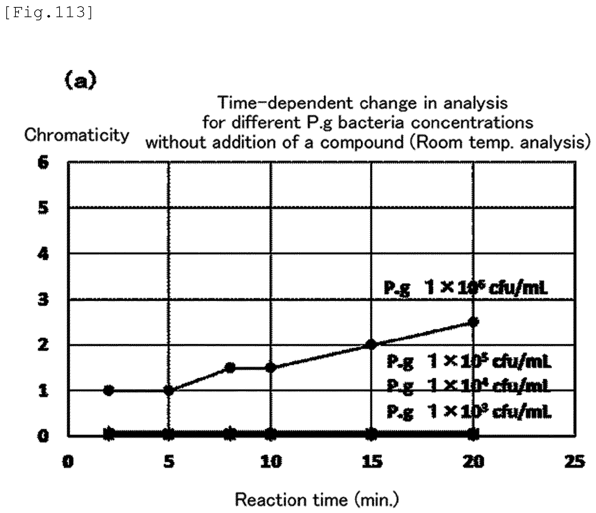

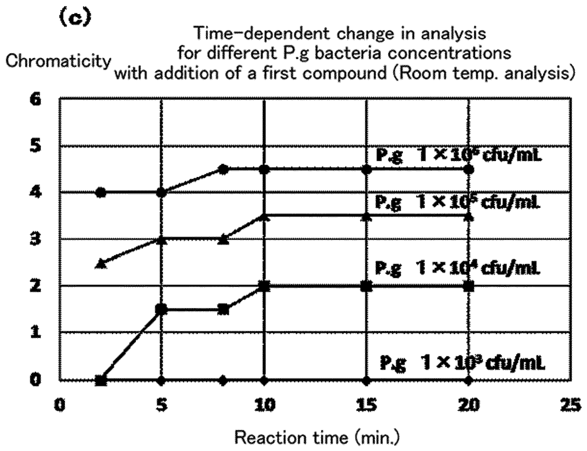

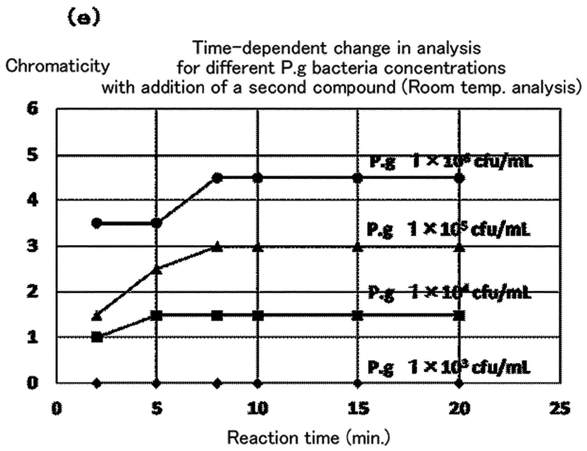

Fig. 113(a) to (b) are graphs showing time-dependent change in analysis for different P.g bacteria concentrations in a method of analyzing periodontopathic bacteria using a dry retention method without addition of a first compound or second compound (Comparative Example 1). Fig. 113(c) to (d) are graphs showing time-dependent change in analysis for different P.g bacteria concentrations in a method of analyzing periodontopathic bacteria using a dry retention method with 25 mM L-ascorbic acid (Example 1). Fig. 113(e) to (f) are graphs showing time-dependent change in analysis for different P.g bacteria concentrations in a method of analyzing periodontopathic bacteria using a dry retention method with 25 mM thioglycolic acid (Example 1).

### MODES FOR CARRYING OUT THE INVENTION

Although examples of the present invention shall now be described specifically, these examples do not limit the scope of the present invention.

### (Preparation of first compounds and second compounds)

As compounds (first compounds) having an antioxidant effect, 62.5 mM, 125 mM, 250 mM, 500 mM, and 1000 mM of each of L-ascorbic acid, L-cysteine hydrochloride, and glutathione were sampled, and each of the sampled first compounds was dissolved in 50 mM tris-maleate buffer solution pH 8.5 and thereafter, pH adjustment was performed such that the pH became 8.5.

As compounds (second compounds) having action of protecting SH groups and cleaving disulfide bonds, each of DTT, thioglycolic acid, thioglycerol, mercaptoethanol, and TCEP were dissolved in 50 mM tris-maleate buffer solution pH 8.5 to concentrations of 62.6 mM, 125 mM, 250 mM, 500 mM, and 1000 mM and thereafter, pH adjustment was performed such that the pH became 8.5.

### (Preparation of substrate)

As a substrate, N-α-benzoyl-DL-arginine-2-naphthylamide hydrochloride (obtained from Sigma-Aldrich Japan LLC) was dissolved in 50 mM tris-maleate buffer solution pH 8.5. The substrate concentration is 0.11% by weight.

### (Preparation of coloring solution)

4-(dimethylamino)cinnamaldehyde (DMAC) (obtained from Sigma-Aldrich Japan LLC) was dissolved in 1 mol/L hydrochloric acid. The concentration of DMAC is 0.1% by weight.

### (Culturing of P. g bacteria (sample))

Prepared Porphyromonas gingivalis (P. g bacteria) JCM12257 was inoculated into a broth medium and anaerobically cultured at 37°C for 24 hours and a bacterial culture solution of 1.0 × 10⁷ cfu/mL was obtained.

The broth medium was prepared with 3.0 g of trypticase soy broth, 0.5 g of yeast extract, 0.05 g of L-cysteine hydrochloride, 0.1 mL of hemin solution, 0.02 mL of vitamin K1 solution, and 100 mL of distilled water and after preparation, sterilization at 121°C was performed for 15 minutes.

With the hemin solution, 0.005 g of hemin and 0.0174 g of potassium hydrogen phosphate were dissolved in 1 mL of distilled water. With the vitamin K1 solution, 5 mg of vitamin K1 were dissolved in 1 mL of ethanol.

As a method for measuring bacterial count, the broth culture solution after anaerobic culturing was 10-fold serially diluted with sterilized physiological saline and inoculated onto CDC anaerobe sheep blood agar (obtained from Becton, Dickinson and Co.) and thereafter, anaerobic culturing at 37°C was performed for 48 hours and bacterial count measurement was performed by counting colonies grown on the medium with the naked eye.

### (Example 1) Implementation of dry retention method with addition of a first compound or a second compound alone

### (A) Dry retention of substrate and first compound or second compound

A prepared first compound or second compound and the substrate were mixed at a ratio of 1:9, and 10 mm paper disks (obtained from Advantec Co., Ltd.) were impregnated with 80 µL of the mixture per disk. The paper disks were then dried overnight at 25°C.

### (B) Preparation of P. g

The bacterial culture solution after 24 hours of culturing was 10-fold serially diluted with 50 mM tris-maleate buffer solution pH 8.5 to prepare bacterial suspensions of 1.0 × 10⁶ cfu/mL, 1.0 × 10⁵ cfu/mL, 1.0 × 10⁴ cfu/mL, and 1.0 × 10³ cfu/mL.

### (C) Test method

Two of the paper disks, impregnated with the first compound or the second compound of each concentration and the substrate and then each impregnated with 80 µL of the prepared P. g bacterial suspension, were prepared and left to stand respectively for 2 minutes, 5 minutes, 8 minutes, 10 minutes, 15 minutes, and 20 minutes, one at room temperature and the other at 50°C. The former shall be referred to as room temperature analysis and the latter as 50°C analysis. Thereafter, 30 µL of the coloring solution was dripped onto each paper disk and coloration of each paper disk was observed and judged with the naked eye.

### (D) Results

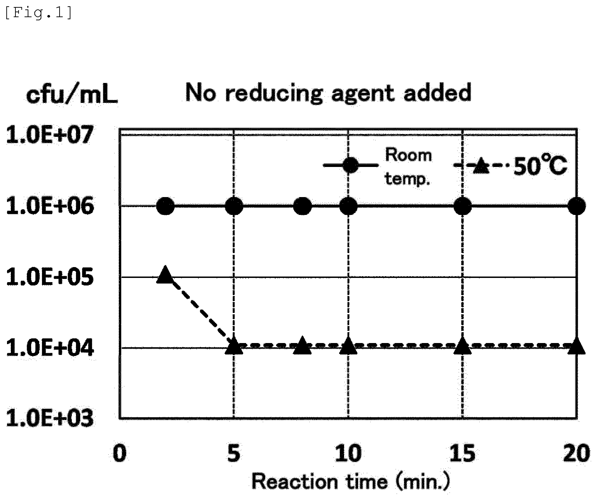

Fig. 1 shows P.g detection sensitivities without addition of a first compound or second compound. As shown in Fig. 1, only 1.0 × 10⁶ cfu/mL could be detected even after 20 minutes in room temperature analysis in the absence of the first compound or second compound, but with 50°C analysis, detection of up to 1.0 × 10⁴ cfu/mL was possible at 5 minutes, indicating that 50°C analysis has a detection sensitivity of 100-fold compared to room temperature, in a short period of time.

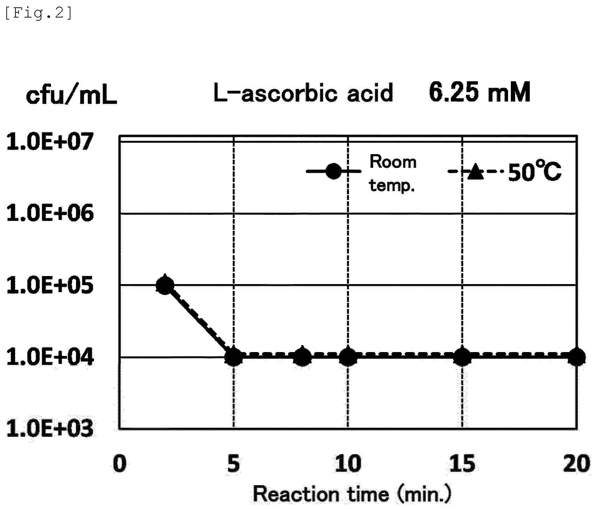

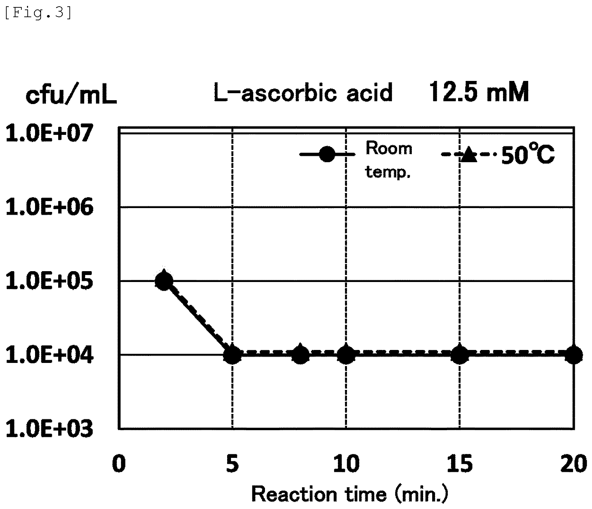

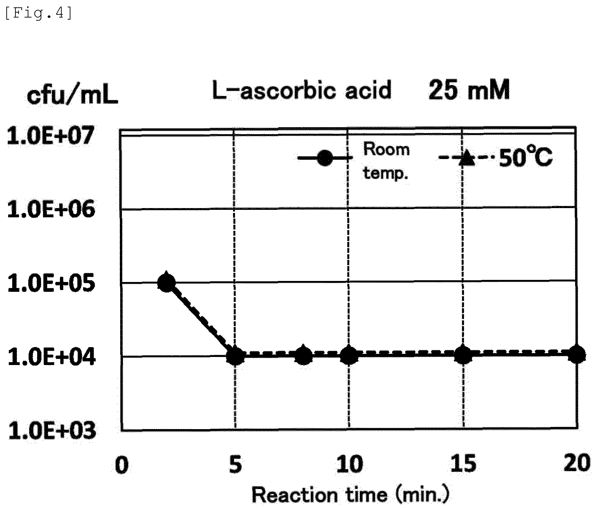

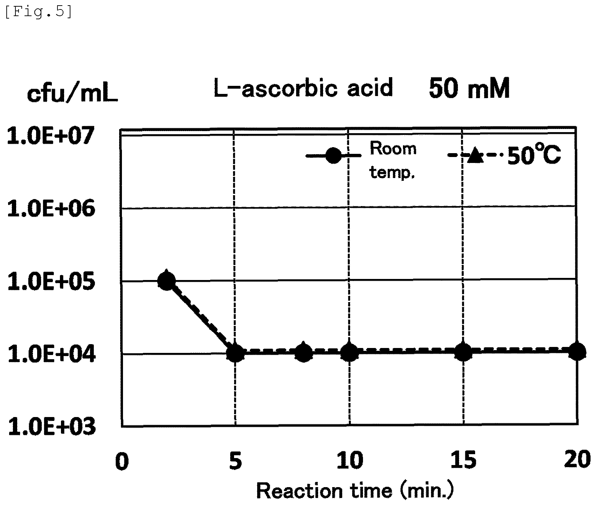

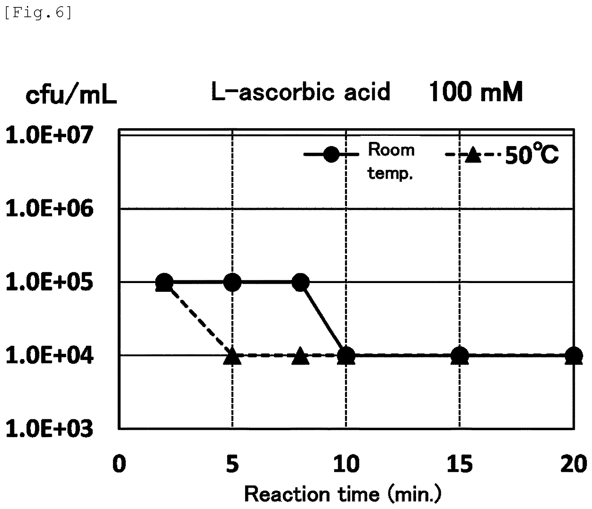

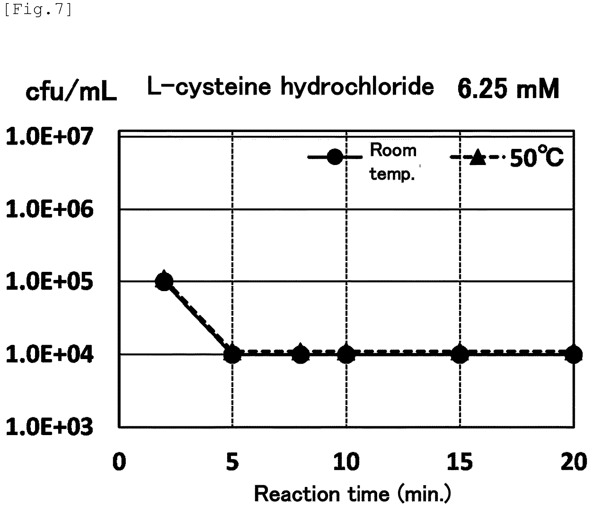

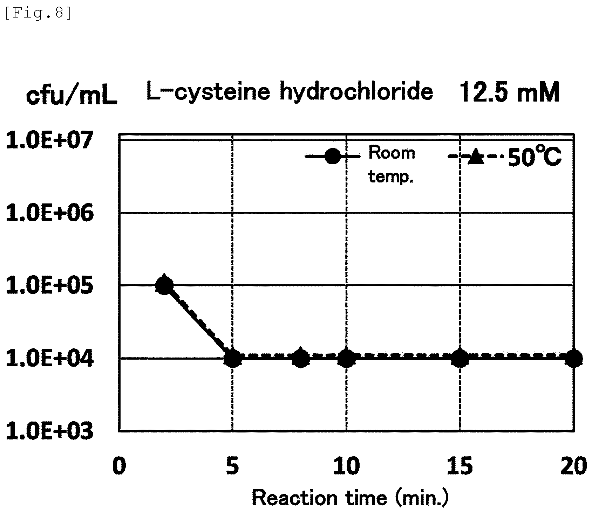

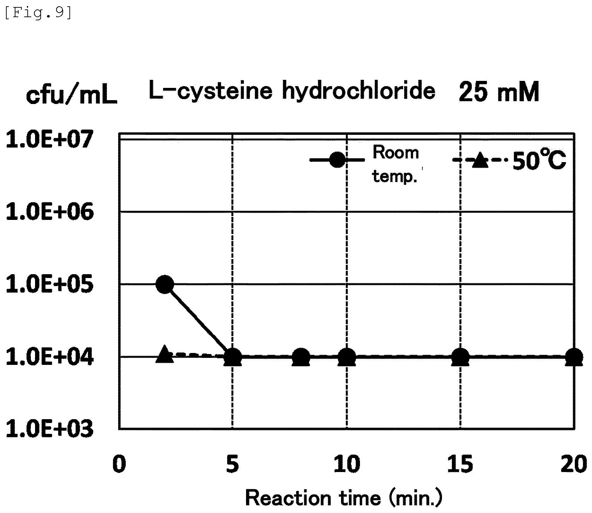

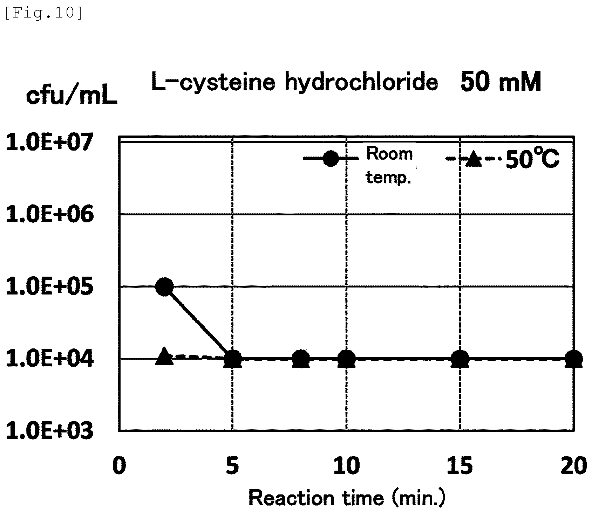

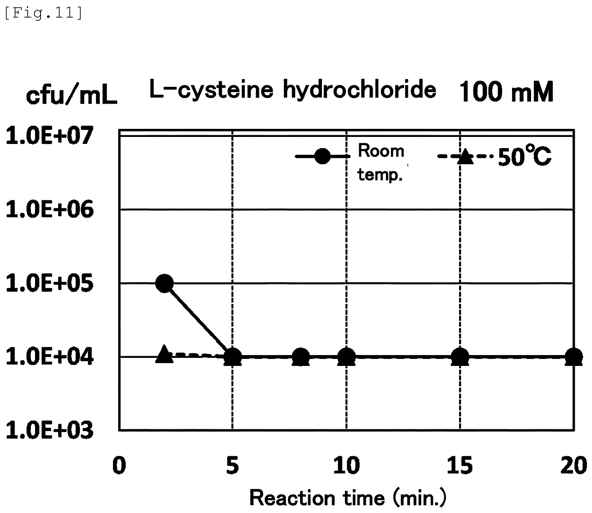

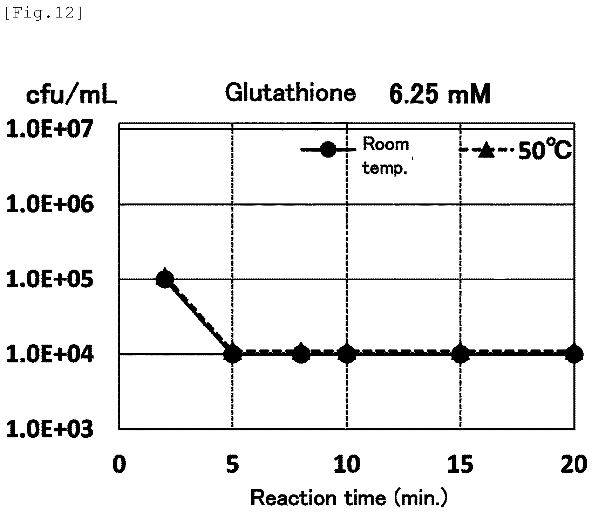

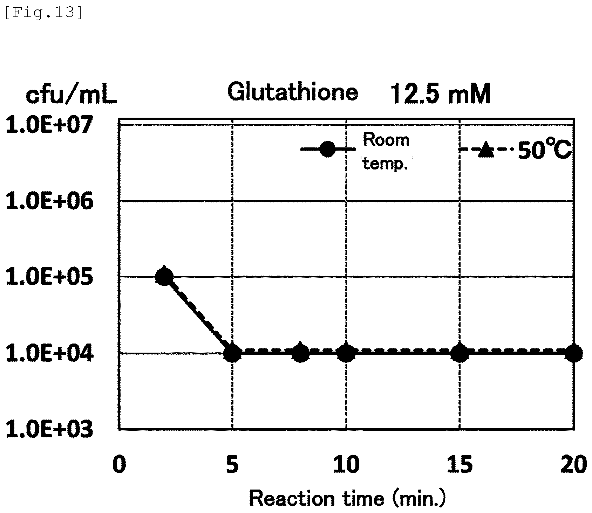

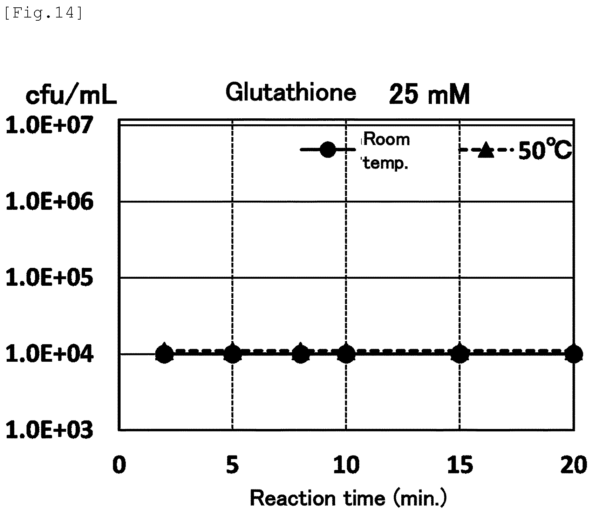

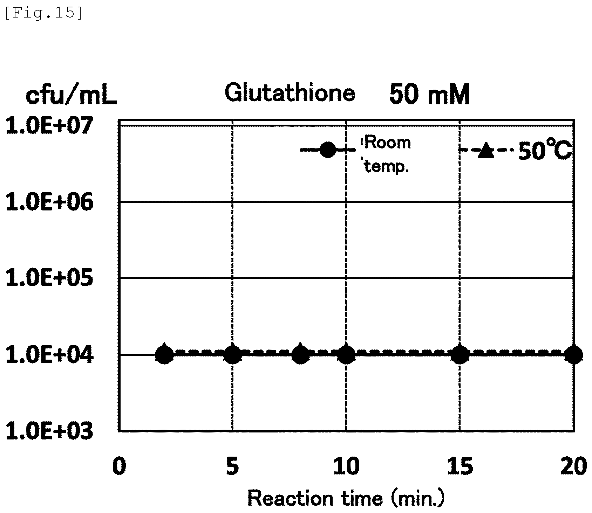

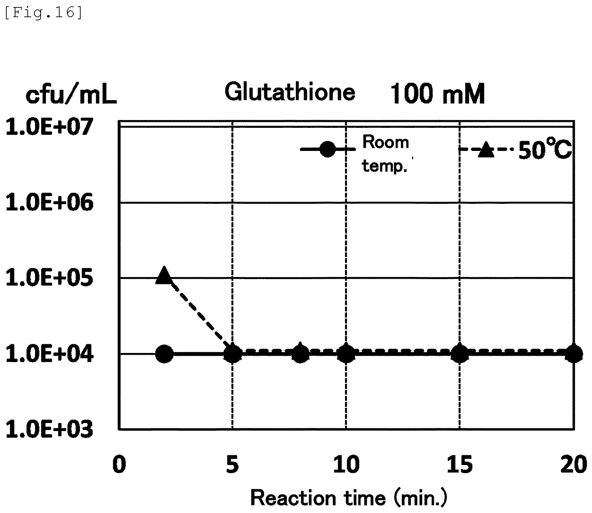

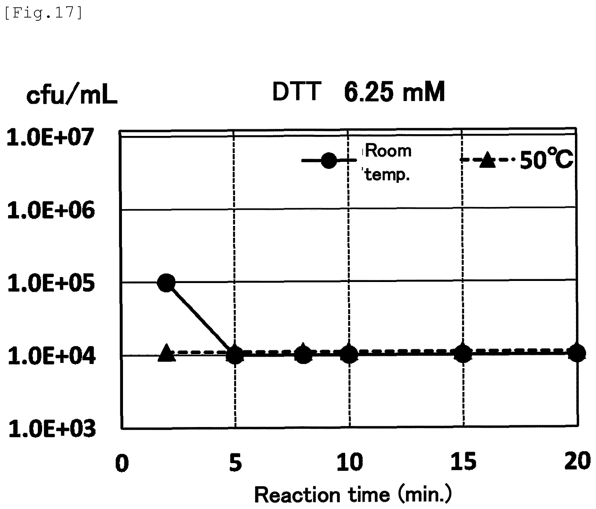

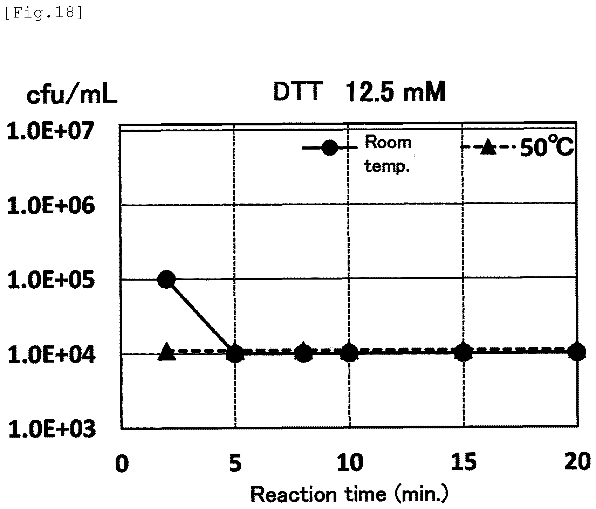

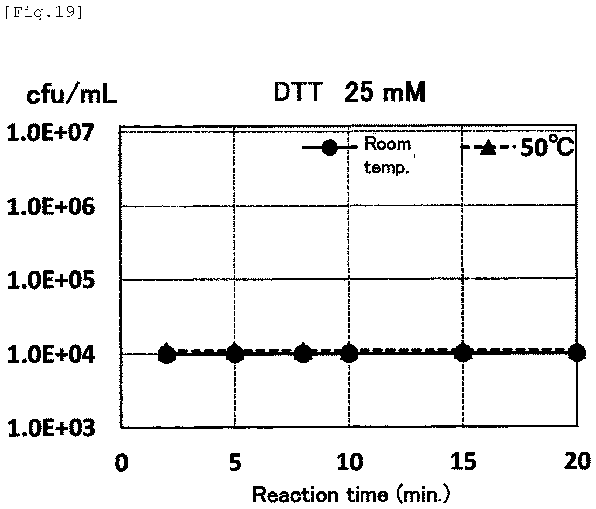

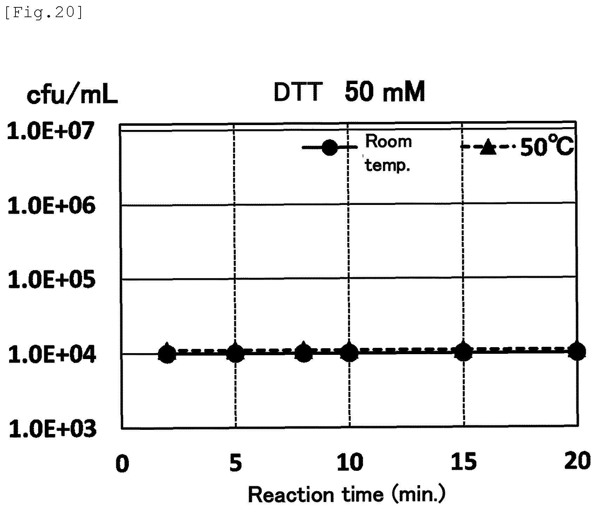

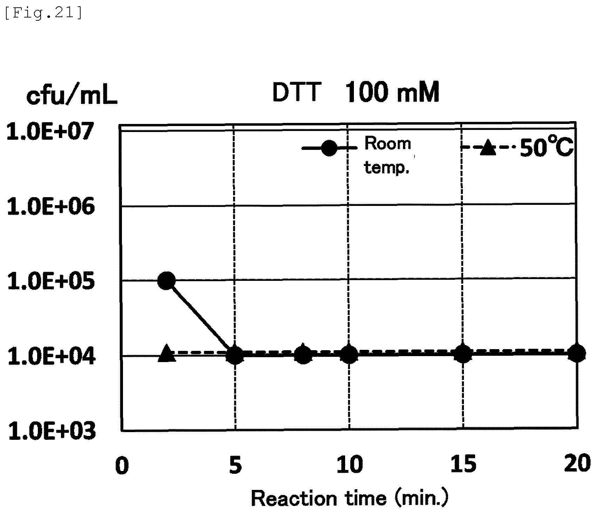

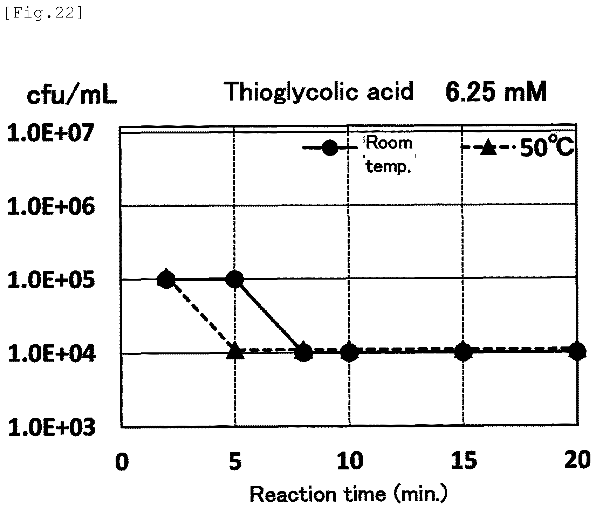

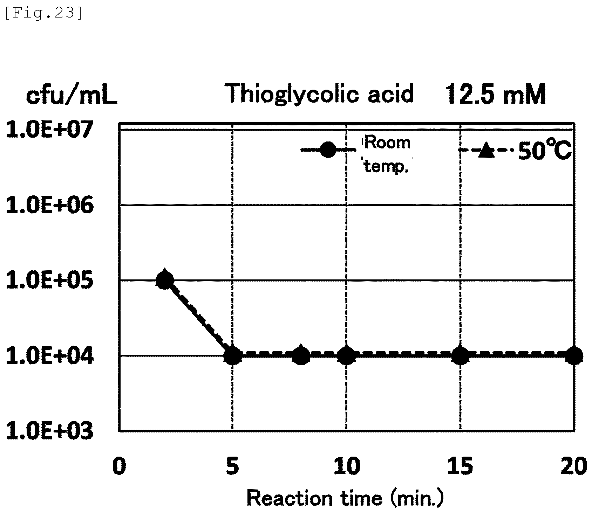

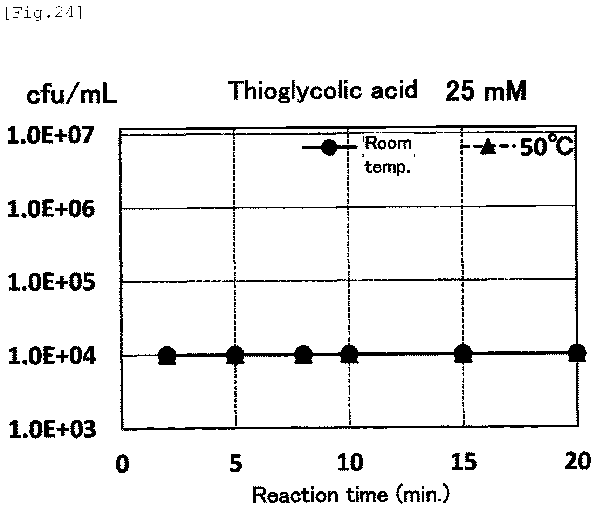

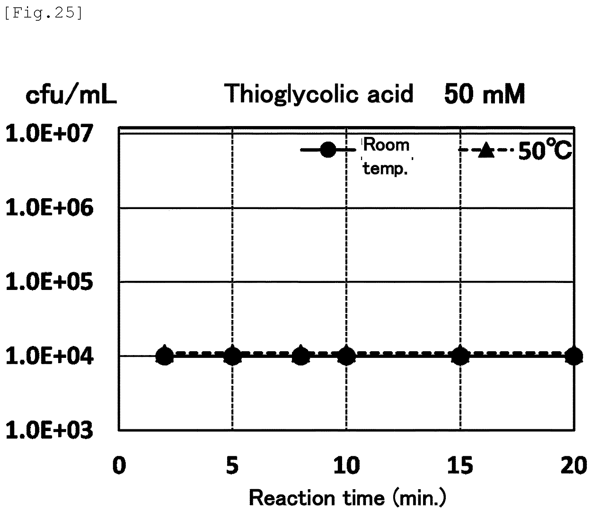

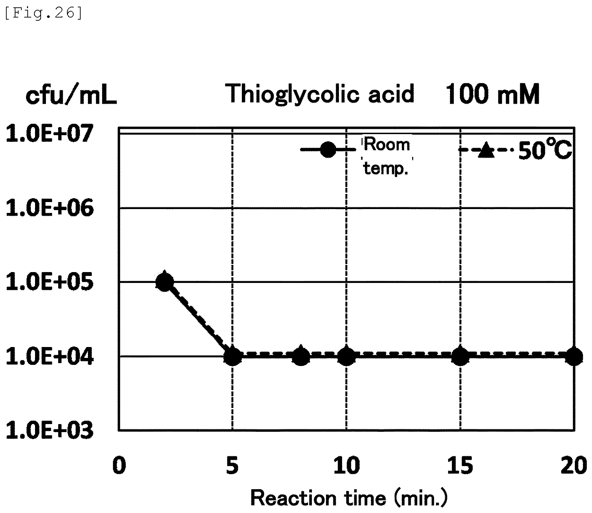

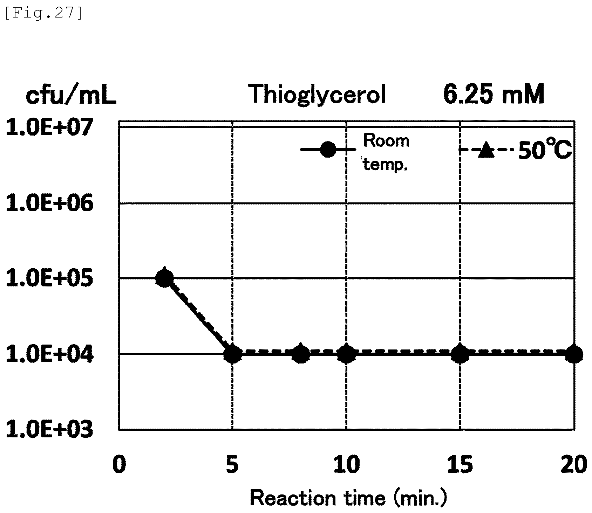

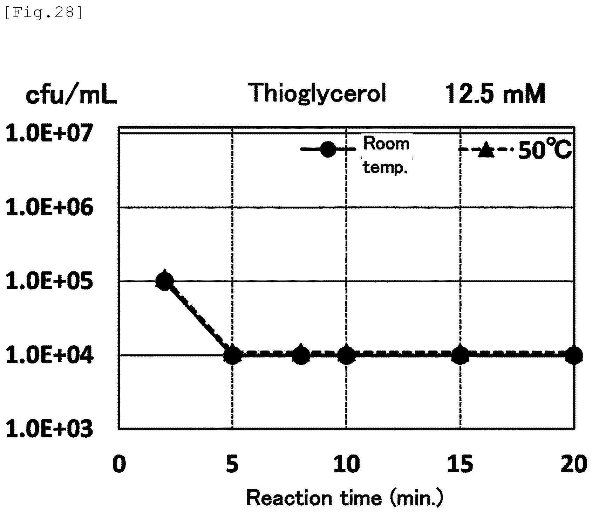

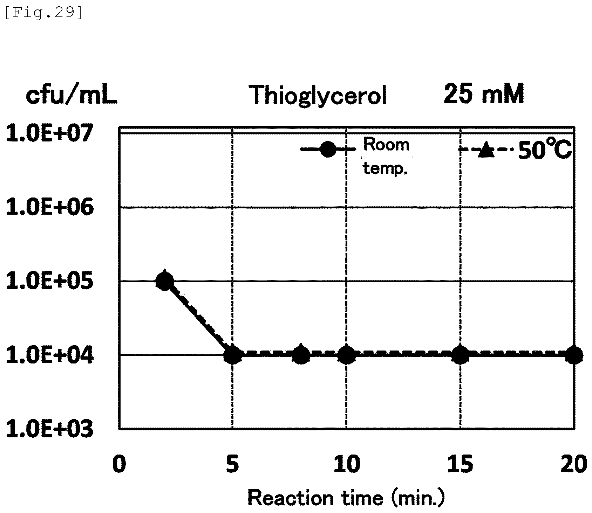

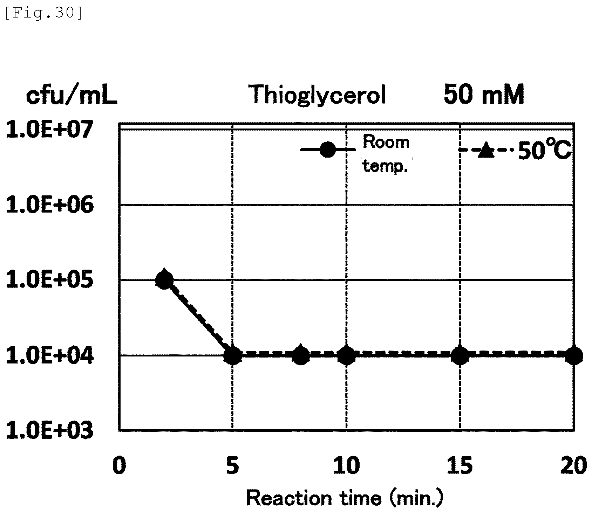

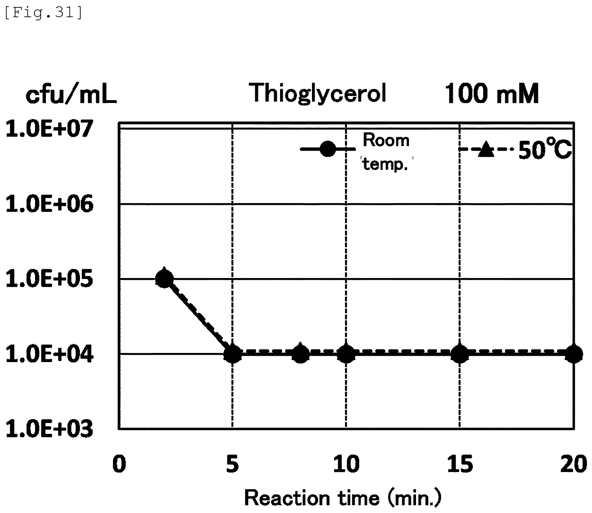

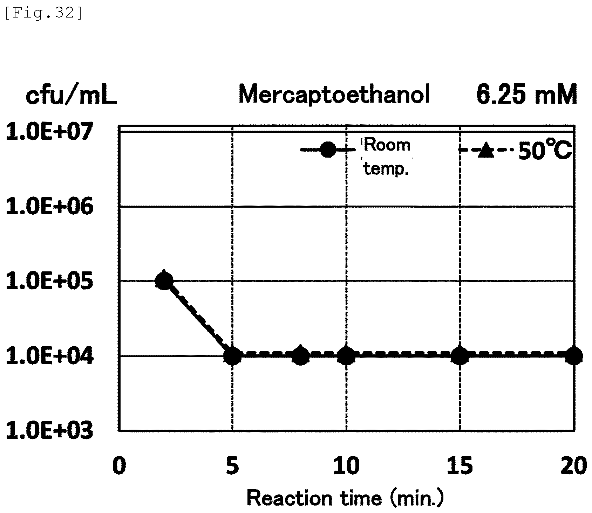

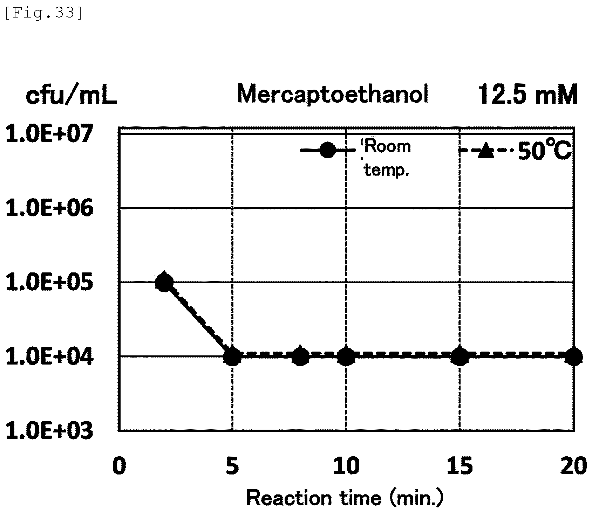

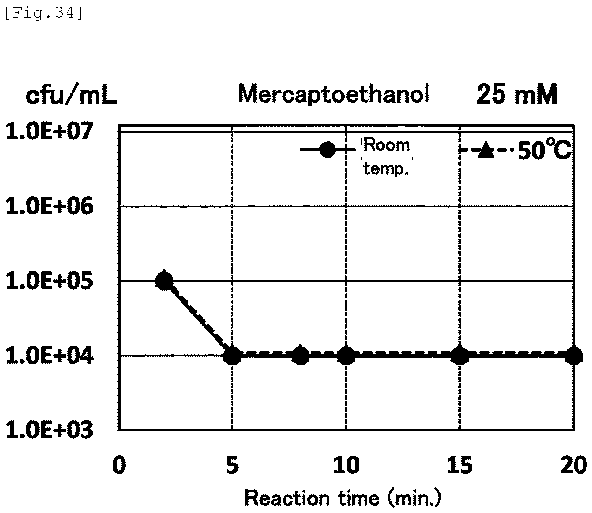

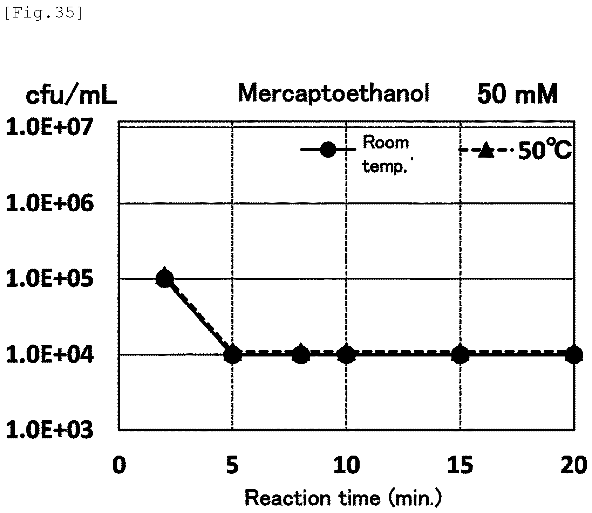

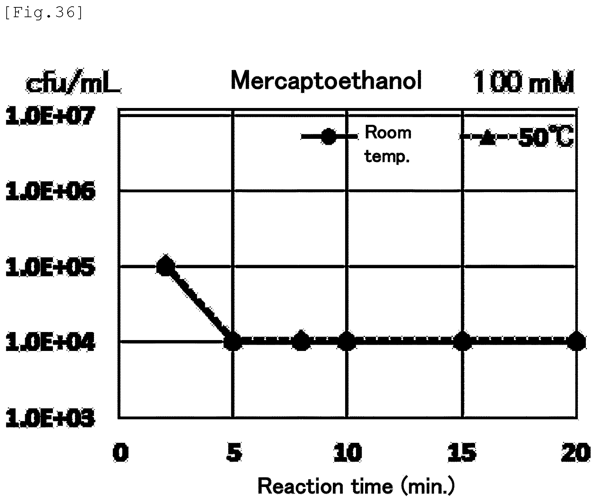

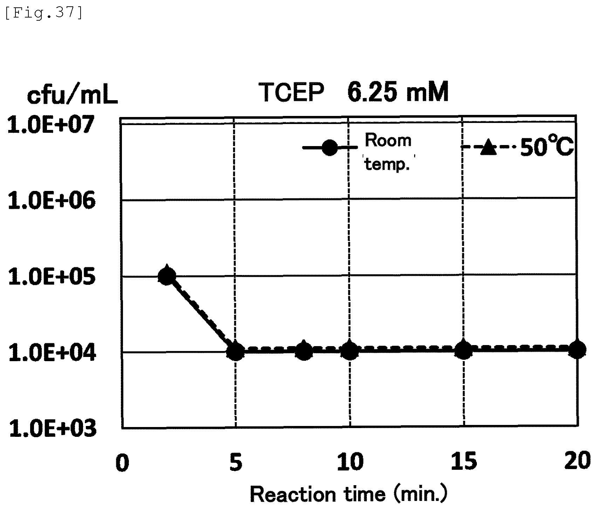

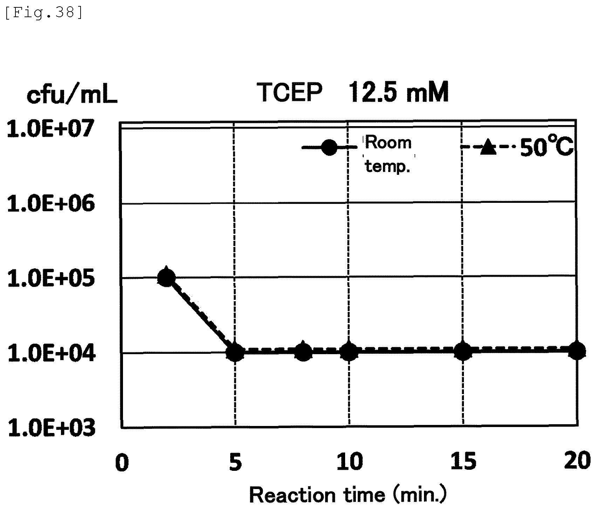

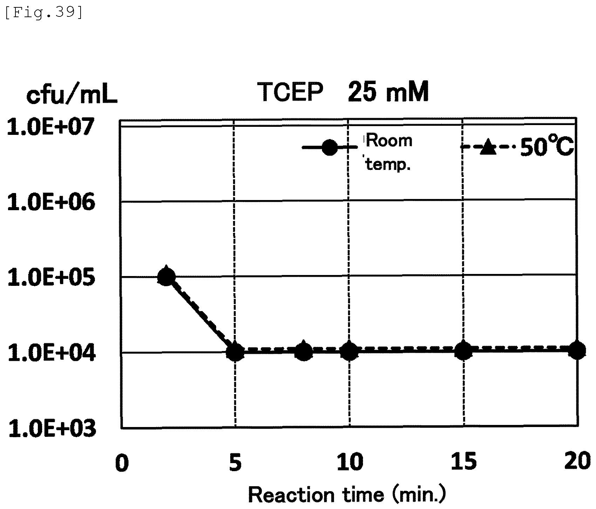

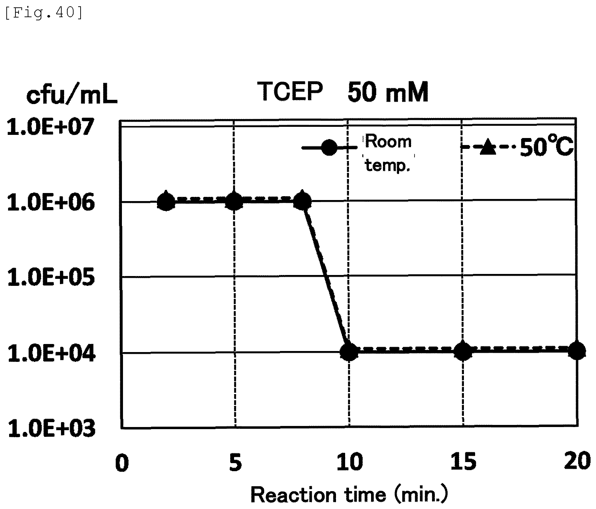

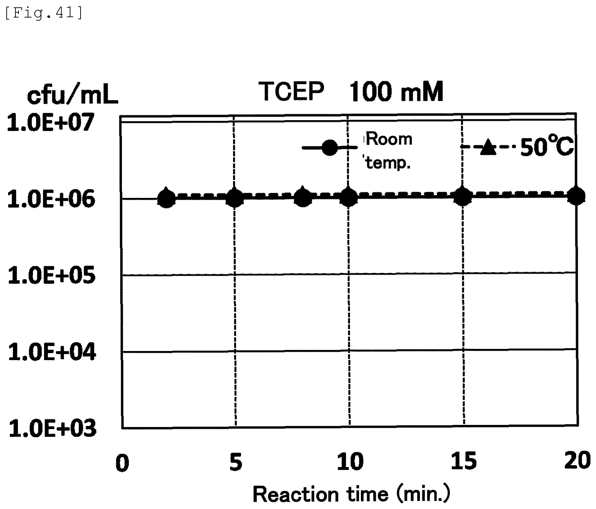

Fig. 2 to Fig. 6 show detection times and detection sensitivities in the presence of L-ascorbic acid as the first compound. Fig. 7 to Fig. 11 show detection times and detection sensitivities in the presence of L-cysteine hydrochloride as the first compound. Fig. 12 to Fig. 16 show detection times and detection sensitivities in the presence of glutathione as the first compound. Fig. 17 to Fig. 21 show detection times and detection sensitivities in the presence of DTT as the second compound. Fig. 22 to Fig. 26 show detection times and detection sensitivities in the presence of thioglycolic acid as the second compound. Fig. 27 to Fig. 31 show detection times and detection sensitivities in the presence of thioglycerol as the second compound. Fig. 32 to Fig. 36 show detection times and detection sensitivities in the presence of mercaptoethanol as the second compound. Fig. 37 to Fig. 41 show detection times and detection sensitivities in the presence of TCEP as the second compound. It was shown that sensitivity obtained with 50°C analysis is obtained in an equivalent manner in the presence of a first compound or second compound at an analysis time of less than 10 minutes, even with room temperature analysis. In other words, it was shown that a difference in sensitivity of 100-fold was found between room temperature analysis and 50°C analysis in the absence of a first compound or second compound, with the minimum bacterial concentration (P.g at 1 × 10⁴ cfu/mL) being undetectable even at 20 minutes, but that in the presence of a first compound or second compound, with any of the compounds, even with room temperature analysis it is possible to detect the minimum bacterial concentration in a manner equivalent to 50°C analysis in less than 10 minutes, though with some differences in minimum reaction time depending on the type of compound. Table 1 shows a comparison between minimum analysis times allowing detection of the minimum bacterial concentration in the presence of different concentrations of first compounds and second compounds, for room temperature analysis and 50°C analysis. As shown in Table 1, it was confirmed that the effects of the first compound and second compound had wide optimal concentrations, though with differences in minimum analysis time depending on the types and concentrations of the compounds, and detection of the minimum bacterial concentration was possible in less than 10 minutes at room temperature. Another surprising result was that detection of the minimum bacterial concentration was possible within 2 minutes at room temperature, depending on the types and concentrations of the compounds.

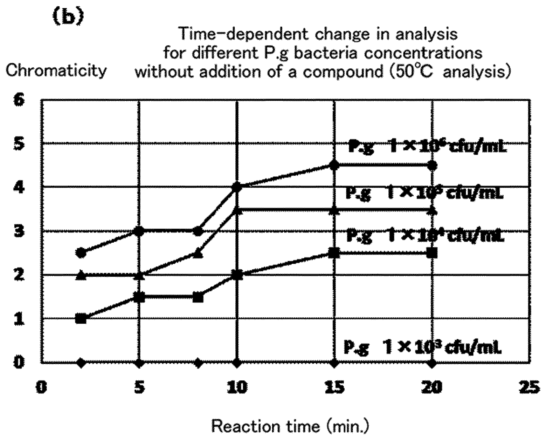

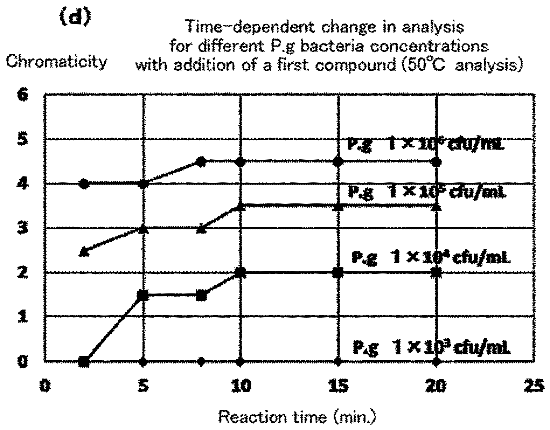

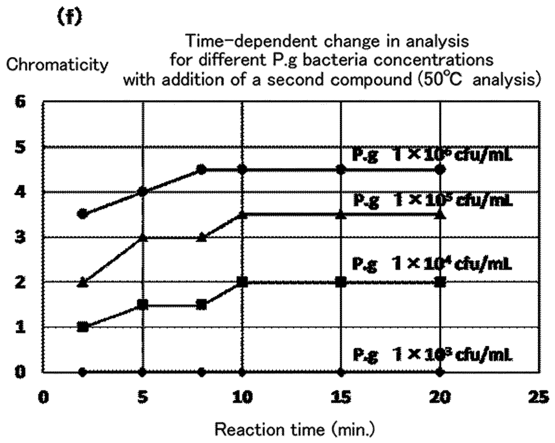

When an enzyme reaction is carried out with a sufficient amount of substrate concentration, the reaction rate changes depending on the concentration of the detection substance, with a higher detection substance concentration allowing the reaction to reach a steady state in a shorter time, and conversely with a lower detection substance concentration requiring more time for the reaction to reach a steady state. In the presence of the first compound and second compound, however, as shown in Fig. 113, it was confirmed that a steady state is reached with a low bacterial concentration in a similar time as with a high concentration. The ordinate of the graph represents color intensity in analysis according to the invention (a higher value representing more stronger color development) and the abscissa represents reaction time. In the absence of the first compound and second compound, reaction was only observed at 1 × 10⁶ cfu/mL with room temperature analysis, and a steady state was not reached even after 20 minutes. In the presence of a first compound and second compound, however, reaction was observed with a P.g bacterial concentration of 1 × 10⁴ cfu/mL even with room temperature analysis, with a steady state being reached at about the same reaction time at all of the bacterial concentrations. Even with 50°C analysis, it was also confirmed that a steady state was reached earlier in the presence of a first compound and second compound than in their absence. In other words, it was demonstrated that the presence of a first compound and second compound allows a steady state to be reached at about the same time at any bacterial concentration, even with room temperature analysis.

**[Table 1]**

| Type of compound | | Room temperature analysis | | | | | | 50°C analysis | | | | | |
|---|---|---|---|---|---|---|---|---|---|---|---|---|---|
| | | Compound concentration mM | | | | | | | | | | | |
| | | 0 | 6.25 | 12.5 | 25 | 50 | 100 | 0 | 6.25 | 12.5 | 25 | 50 | 100 |
| First compound | L-ascorbic acid | × | 5 min. | 5 min. | 5 min. | 5 min. | 10 min. | 5 min. | 5 min. | 5 min. | 5 min. | 5 min. | 5 min. |
| | L-cysteine hydrochloride | | 5 min. | 5 min. | 5 min. | 5 min. | 5 min. | | 5 min. | 5 min. | 2 min. | 2 min. | 2 min. |
| | Glutathione | | 5 min. | 5 min. | 2 min. | 2 min. | 2 min. | | 5 min. | 5 min. | 2 min. | 2 min. | 5 min. |
| Second compound | DTT | | 5 min. | 5 min. | 2 min. | 2 min. | 5 min. | | 2 min. | 2 min. | 2 min. | 2 min. | 2 min. |
| | Thioglycolic acid | | 8 min. | 5 min. | 2 min. | 2 min. | 5 min. | | 5 min. | 5 min. | 2 min. | 2 min. | 5 min. |
| | Thioglycerol | | 5 min. | 5 min. | 5 min. | 5 min. | 5 min. | | 5 min. | 5 min. | 5 min. | 5 min. | 5 min. |
| | Mercaptoethanol | | 5 min. | 5 min. | 5 min. | 5 min. | 5 min. | | 5 min. | 5 min. | 5 min. | 5 min. | 5 min. |
| | TCEP | | 5 min. | 5 min. | 5 min. | 10 min. | × | | 5 min. | 5 min. | 5 min. | 10 min. | × |

| | | | | | | | | | | | | | |
|---|---|---|---|---|---|---|---|---|---|---|---|---|---|
| ×: 1 × 10⁴ cfu/mL undetectable | | | | | | | | | | | | | |

### (Example 2) Implementation of solution method with addition of a first compound or a second compound alone

### (A) Dry retention of substrate

The prepared substrate was mixed in 50 mM tris-maleate buffer solution pH 8.5 such that a volume ratio would be 9:1 and 10 mm paper disks (obtained from Advantec Co., Ltd.) were impregnated with 80 µL of the mixture per disk. The paper disks were then dried overnight at 25°C.

### (B) Preparation of P.g bacteria

The prepared first compound or second compound was diluted 9-fold with 50 mM tris-maleate buffer solution pH 8.5 to obtain a diluted solution of the compound.

The bacterial culture solution after 24 hours of culturing was 10-fold serially diluted with the diluted compound solutions to prepare bacterial suspensions of 1.0 × 10⁶ cfu/mL, 1.0 × 10⁵ cfu/mL, 1.0 × 10⁴ cfu/mL, and 1.0 × 10³ cfu/mL.

### (C) Test method

Two of the paper disks, impregnated with the substrate and then impregnated with 80 µL of the prepared P. g bacterial suspension, were prepared and left to stand respectively for 2 minutes, 5 minutes, 8 minutes, 10 minutes, 15 minutes, and 20 minutes, one at room temperature and the other at 50°C. The former shall be referred to as room temperature analysis and the latter as 50°C analysis. Thereafter, 30 µL of the coloring solution was dripped onto each paper disk and coloration of each paper disk was observed and judged with the naked eye.

### (D) Results

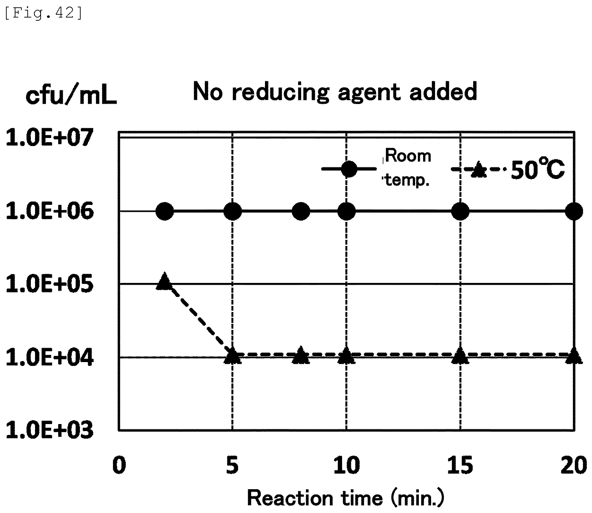

Fig. 42 shows P.g detection sensitivities without addition of the first compound or second compound. As shown in Fig. 42, only 1.0 × 10⁶ cfu/mL could be detected even after 20 minutes in room temperature analysis in the absence of the first compound or second compound, but with 50°C analysis, detection of up to 1.0 × 10⁴ cfu/mL was possible at 5 minutes, indicating that 50°C analysis has a detection sensitivity of 100-fold compared to room temperature, in a short period of time.

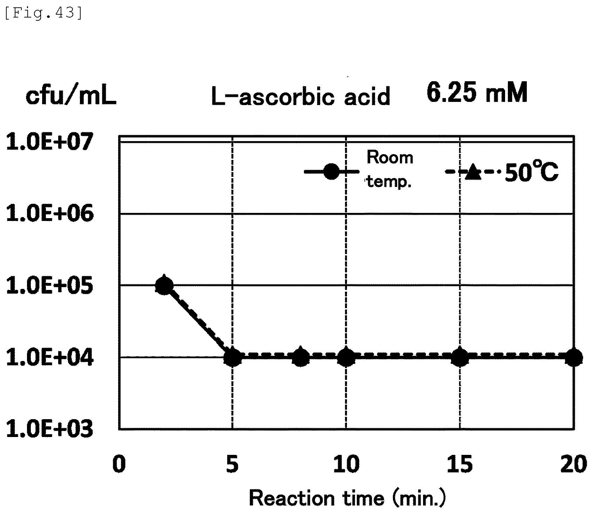

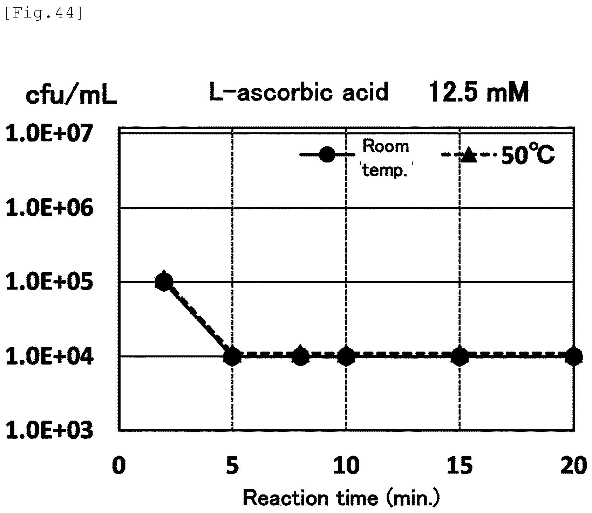

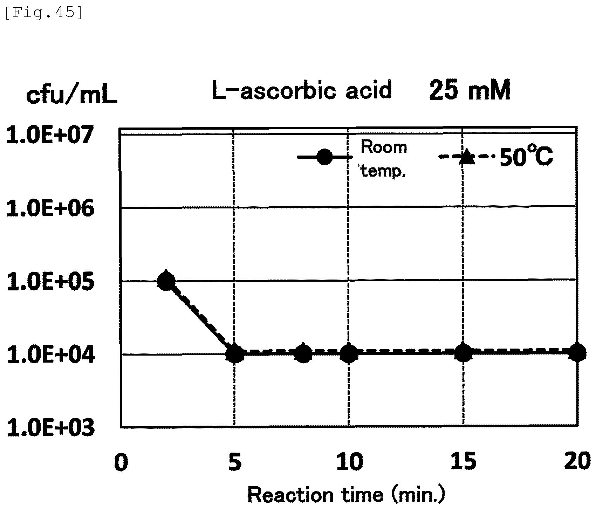

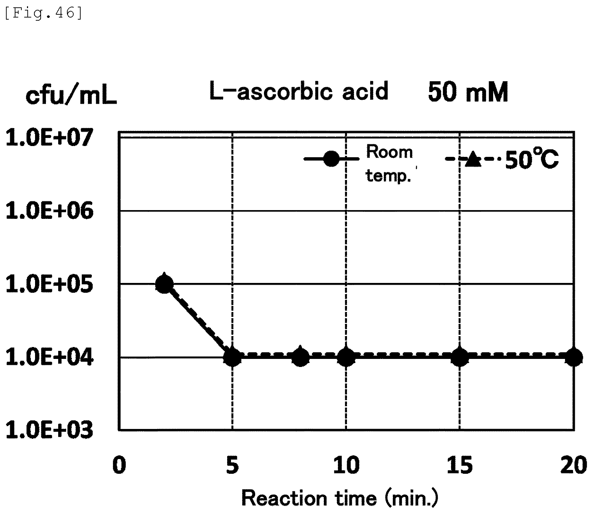

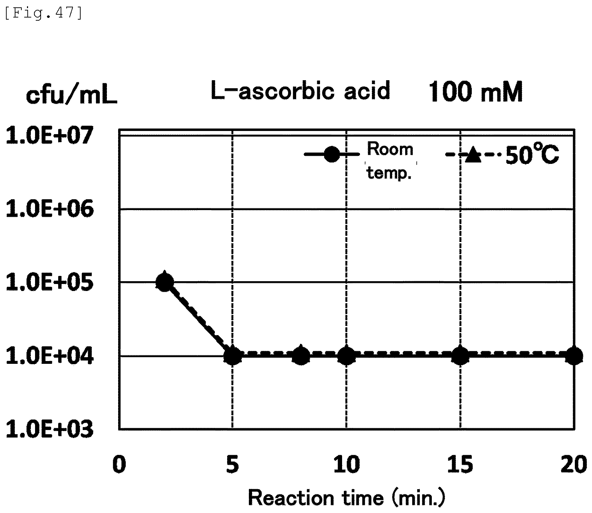

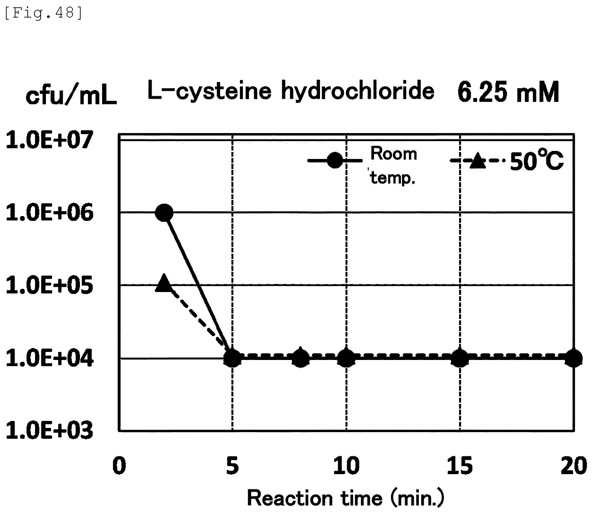

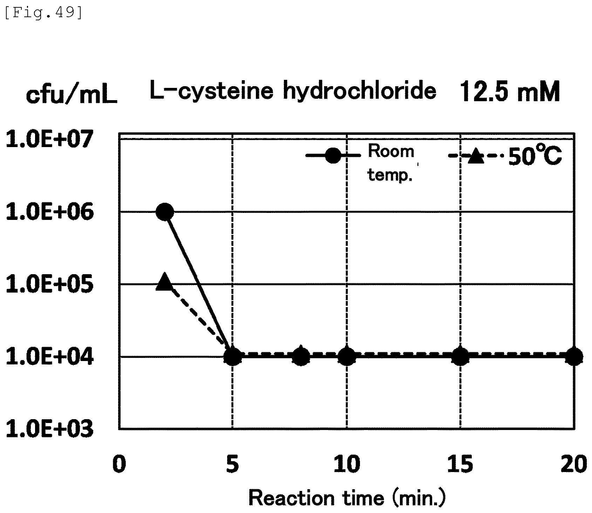

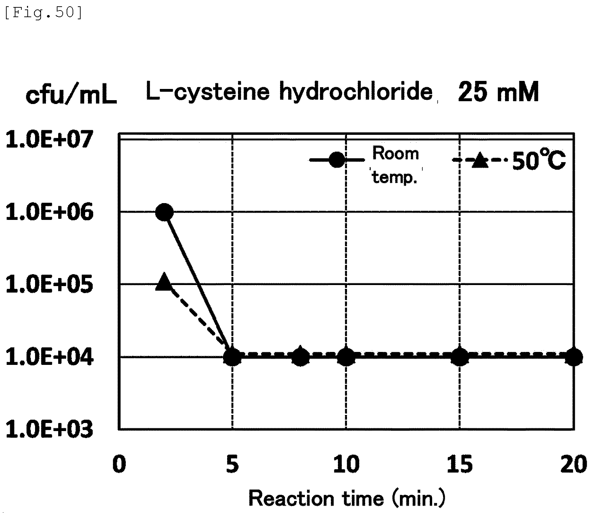

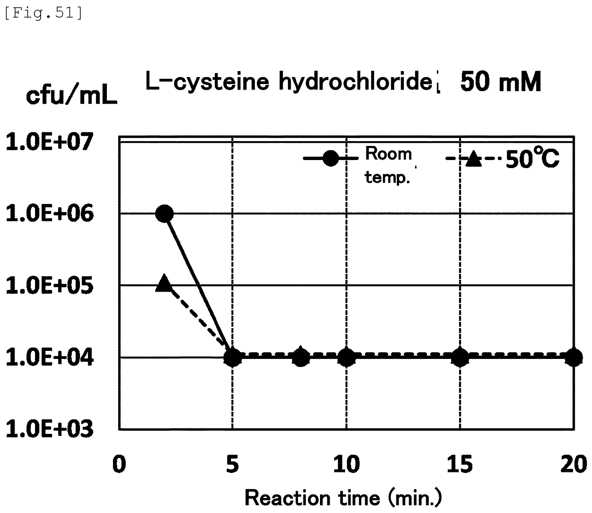

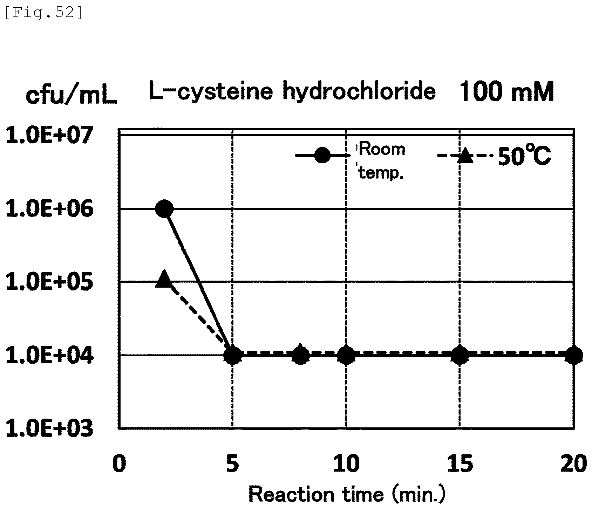

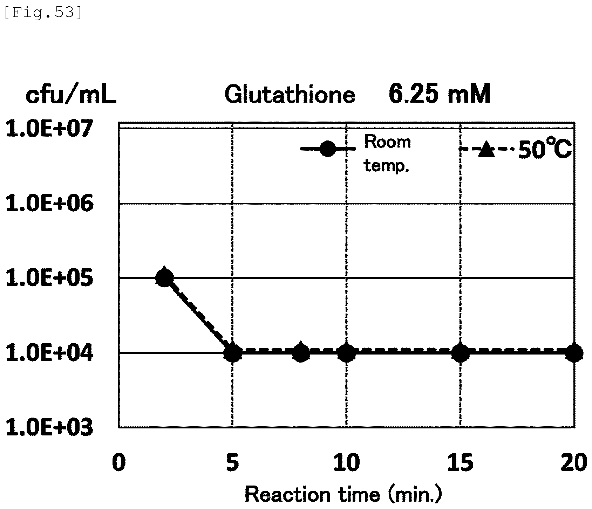

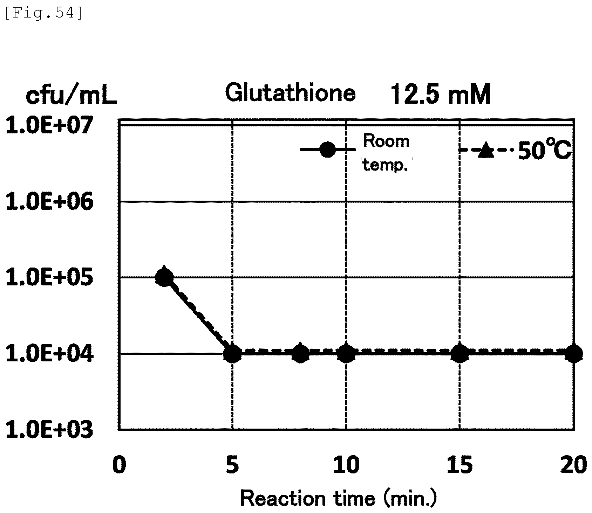

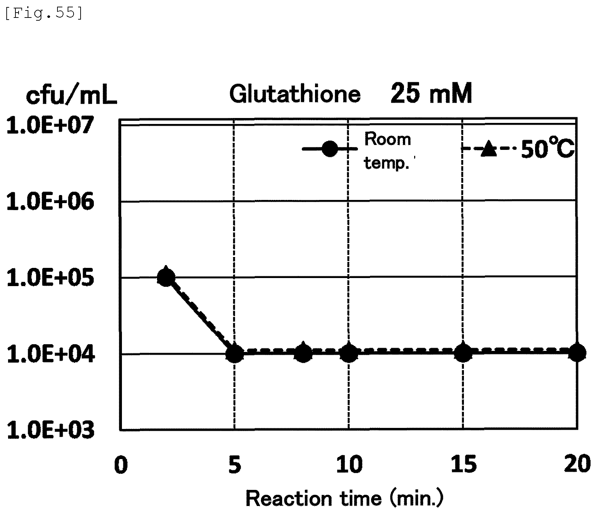

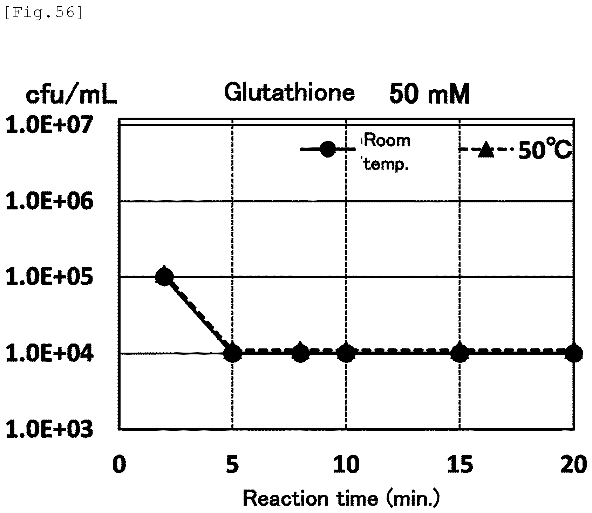

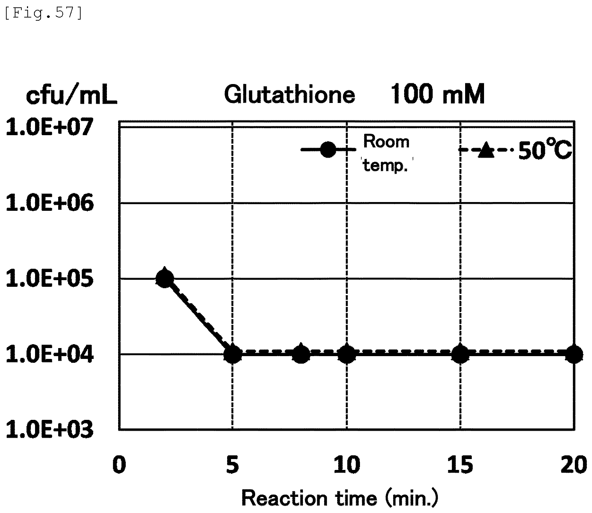

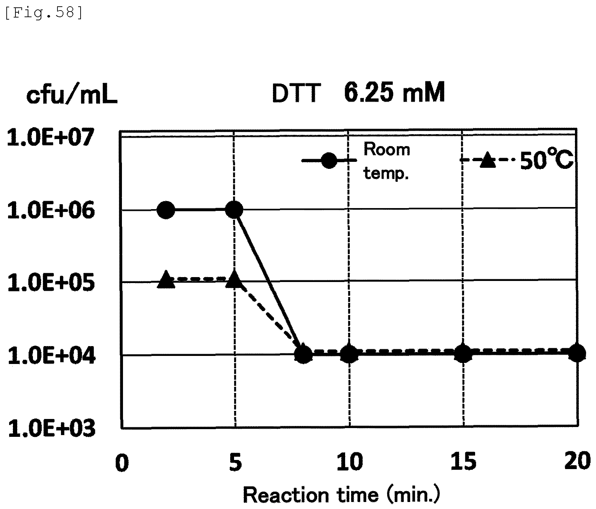

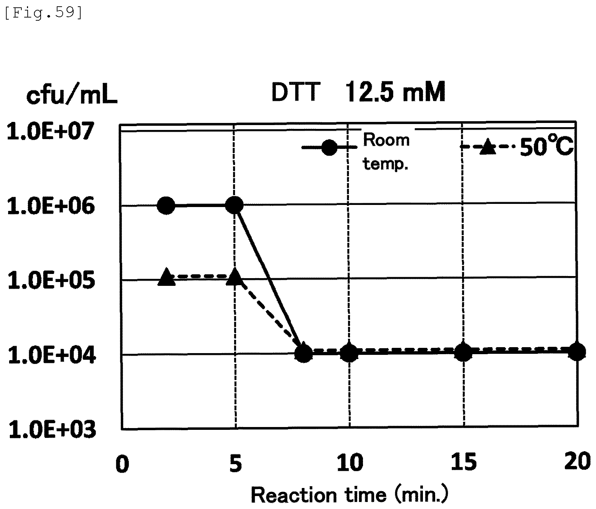

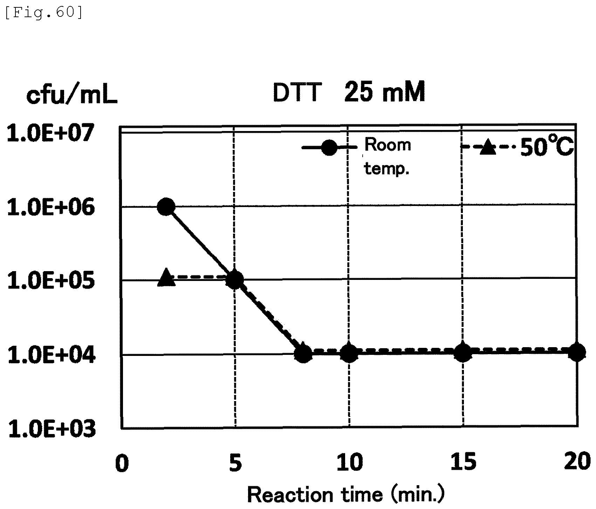

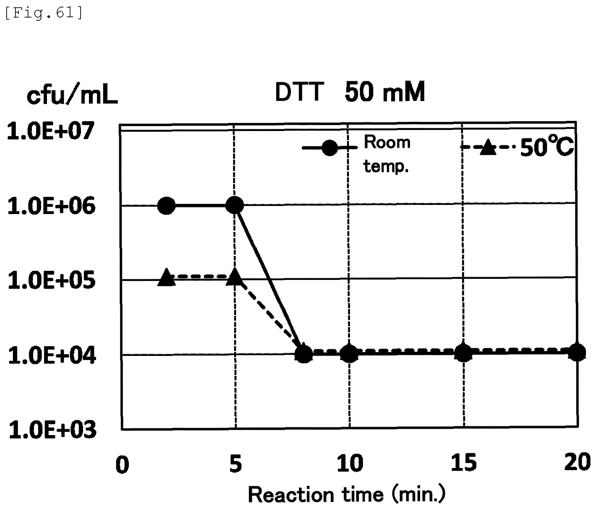

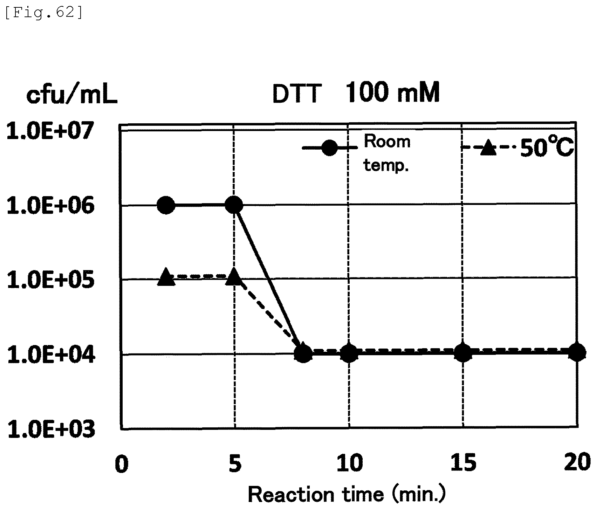

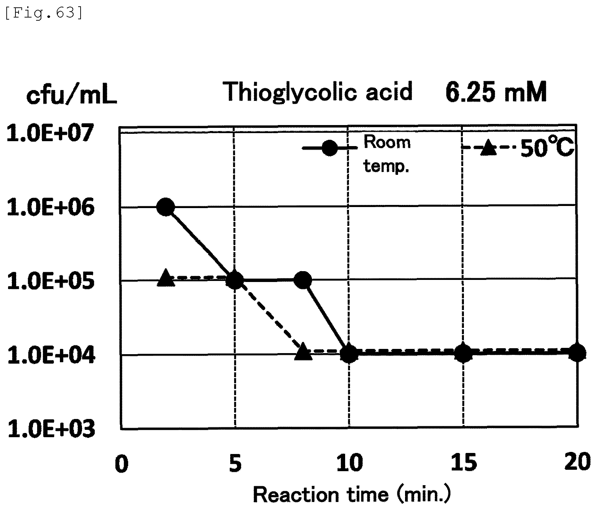

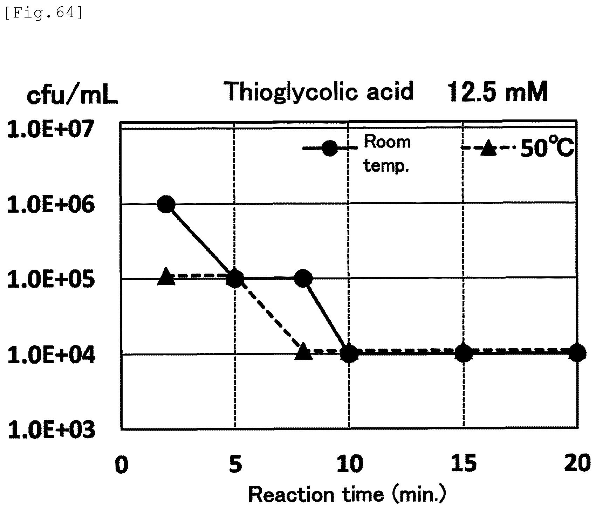

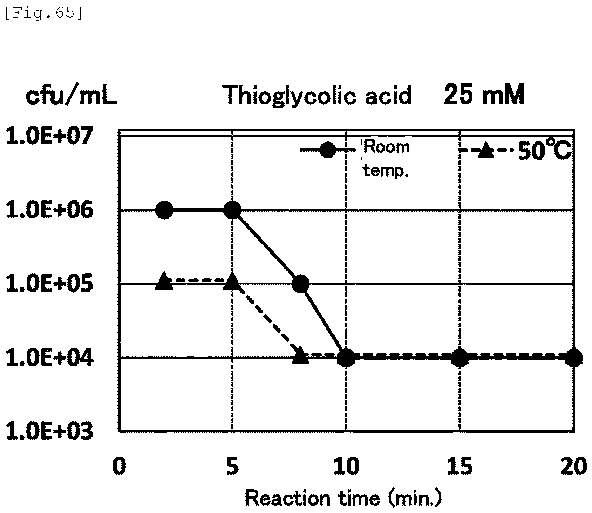

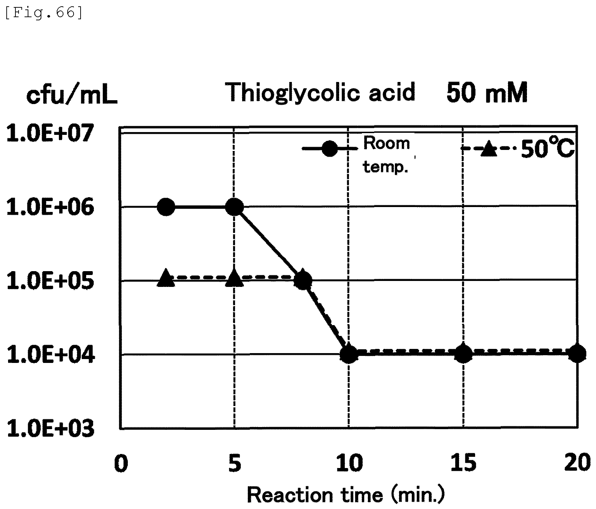

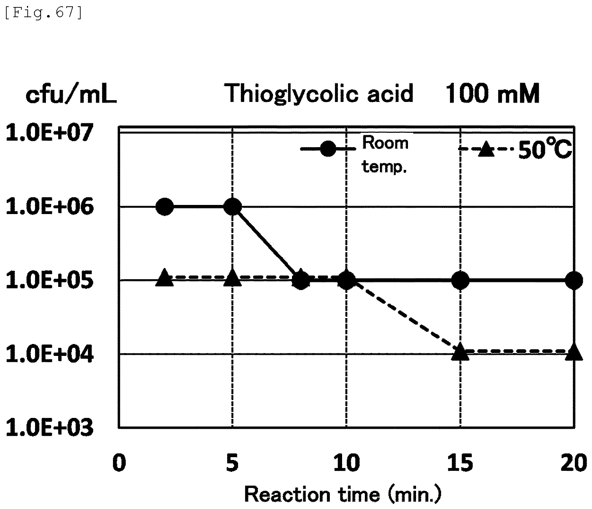

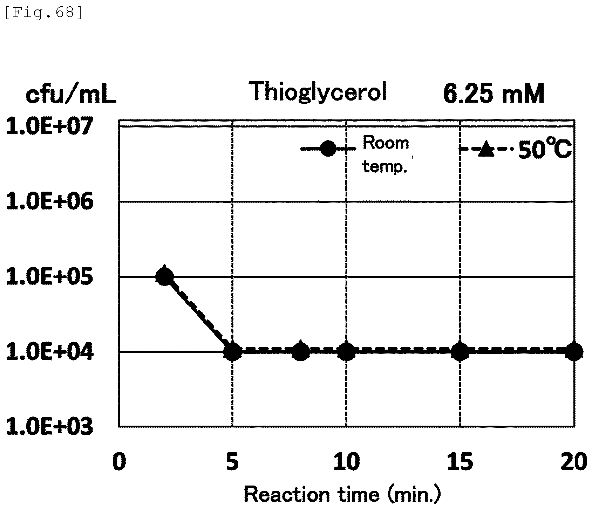

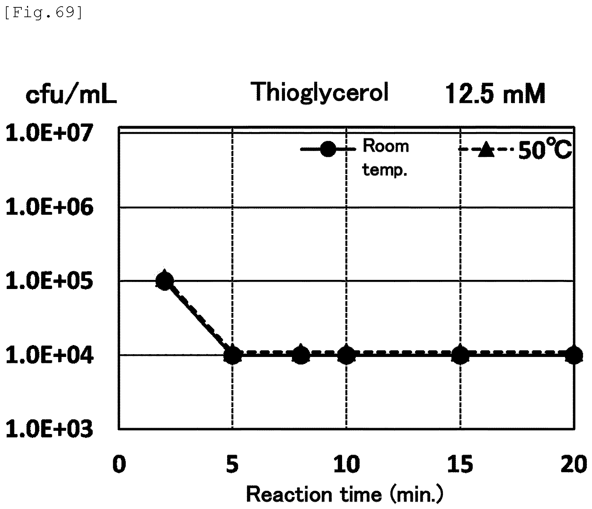

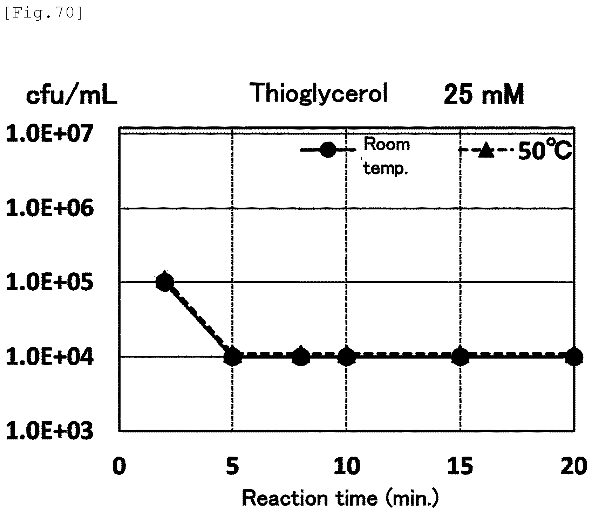

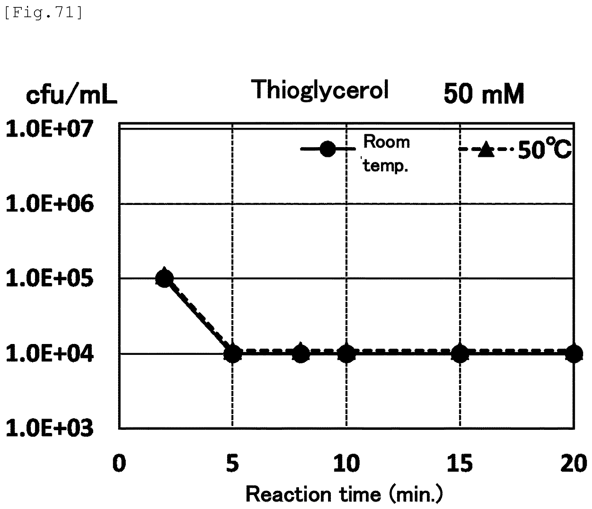

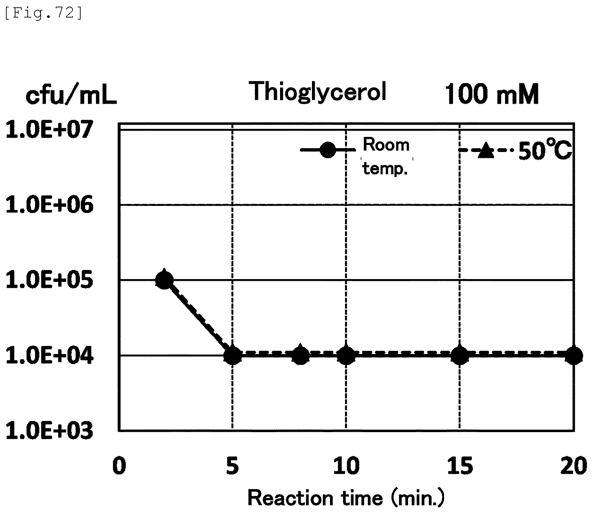

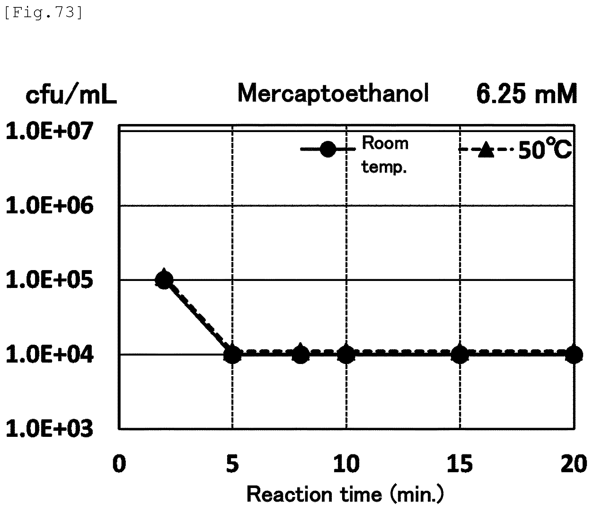

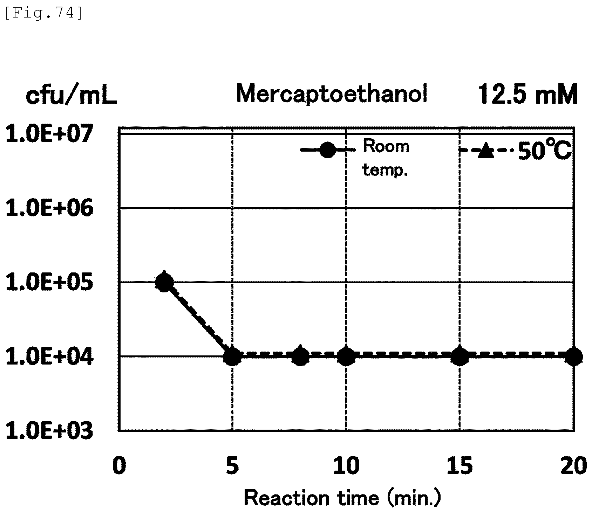

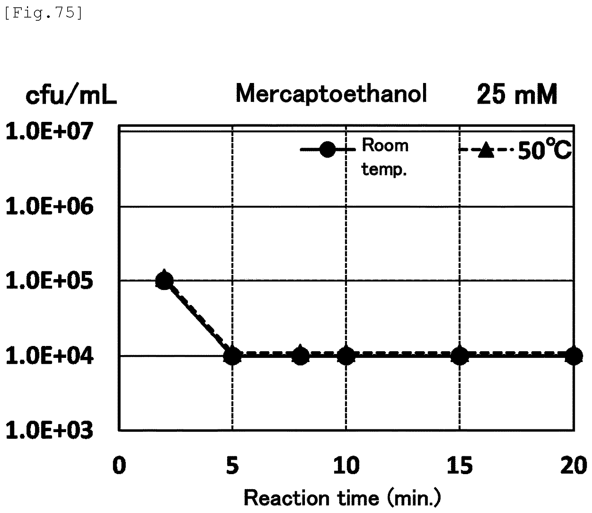

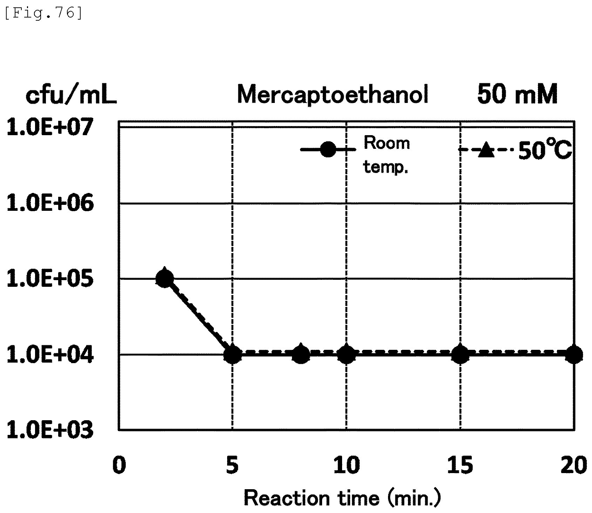

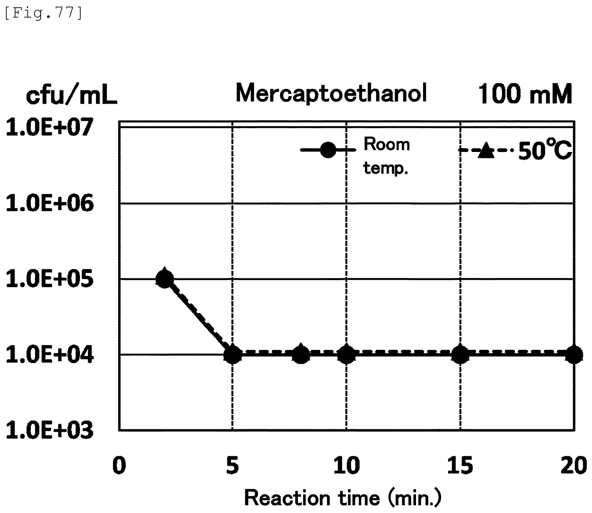

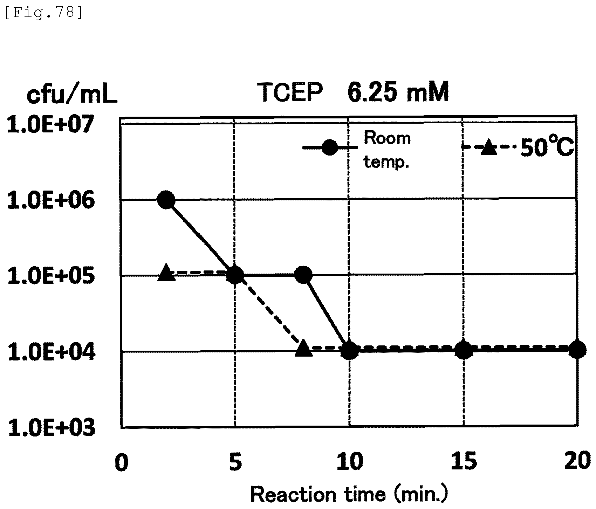

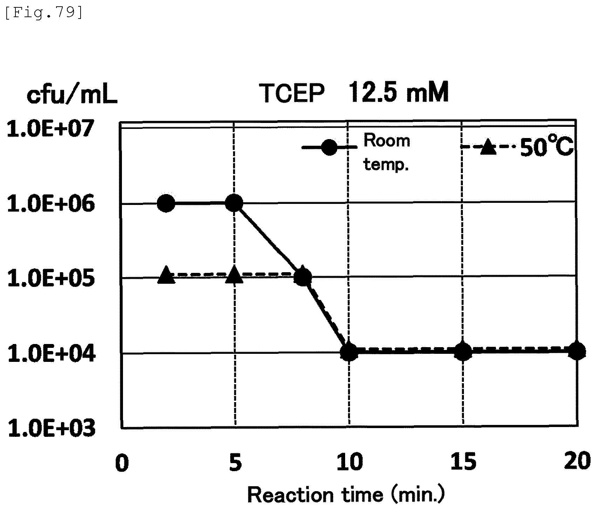

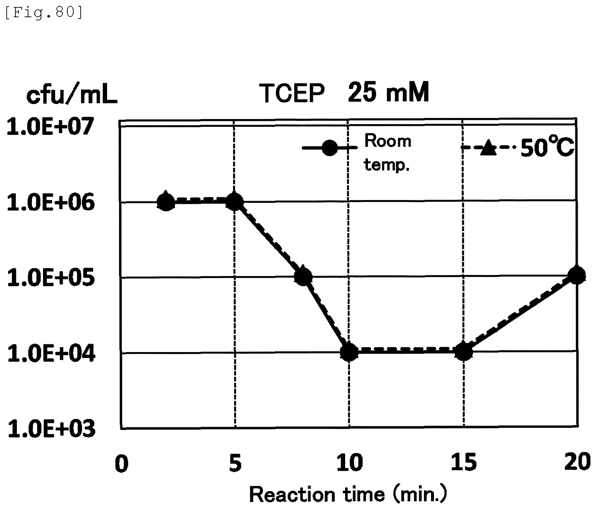

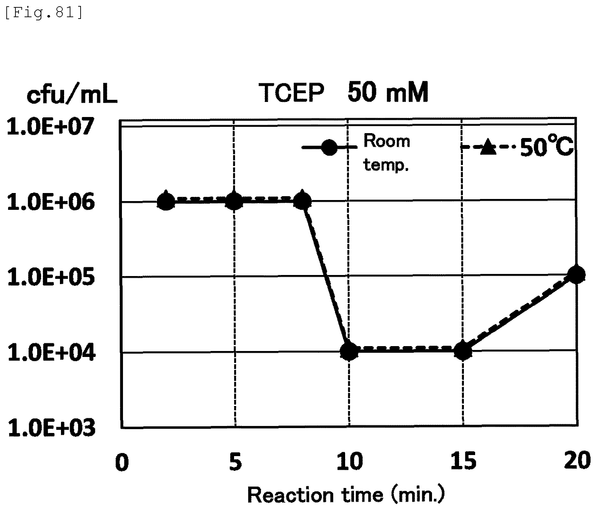

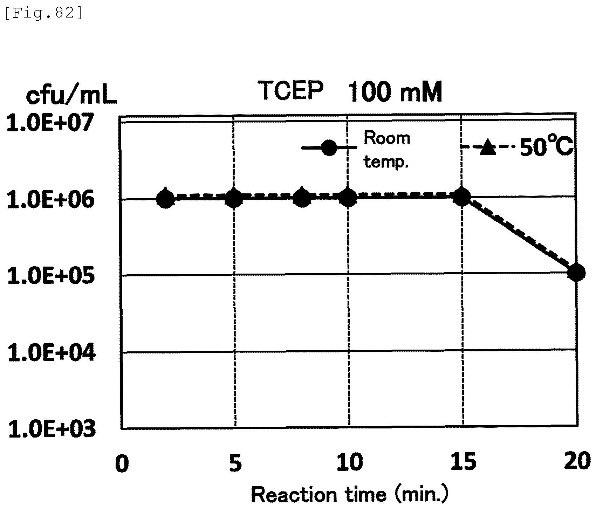

Fig. 43 to Fig. 47 show detection times and detection sensitivities in the presence of L-ascorbic acid as the first compound. Fig. 48 to Fig. 52 show detection times and detection sensitivities in the presence of L-cysteine hydrochloride as the first compound. Fig. 53 to Fig. 57 show detection times and detection sensitivities in the presence of glutathione as the first compound. Fig. 58 to Fig. 62 show detection times and detection sensitivities in the presence of DTT as the second compound. Fig. 63 to Fig. 67 show detection times and detection sensitivities in the presence of thioglycolic acid as the second compound. Fig. 68 to Fig. 72 show detection times and detection sensitivities in the presence of thioglycerol as the second compound. Fig. 73 to Fig. 77 show detection times and detection sensitivities in the presence of mercaptoethanol as the second compound. Fig. 78 to Fig. 82 show detection times and detection sensitivities in the presence of TCEP as the second compound.

Similar to the results for the dry retention method in Example 1, it was shown that in the presence of a first compound or second compound, sensitivity obtained with 50°C analysis is obtained in an equivalent manner at a reaction time of less than 10 minutes, even at room temperature. In other words, it was shown that a difference in sensitivity of 100-fold was found between room temperature analysis and 50°C analysis in the absence of a first compound or second compound, with the minimum bacterial concentration (P.g at 1 × 10⁴ cfu/mL) being undetectable even at 20 minutes, but that in the presence of a first compound or second compound, with any of the compounds, even with room temperature analysis it is possible to detect the minimum bacterial concentration in a manner equivalent to 50°C analysis in less than 10 minutes, though with some differences in minimum analysis time depending on the type of compound. Table 2 shows a comparison between minimum analysis times allowing detection of the minimum bacterial concentration in the presence of different concentrations of first compounds and second compounds, for room temperature analysis and 50°C analysis. As shown in Table 2, it was confirmed that the effects of the first compound and second compound had wide optimal concentrations, though with differences in minimum analysis time depending on the types and concentrations of the compounds, and detection of the minimum bacterial concentration was possible in less than 10 minutes at room temperature. With thioglycolic acid and TCEP, however, while it was possible to detect the minimum bacterial concentration in less than 10 minutes by the dry retention method in Example 1, detection was not possible by the solution method. This is believed to be due to the effect of the second compound being added directly to the bacterial culture solution.

**[Table 2]**

| Type of compound | | Room temperature analysis | | | | | | 50°C analysis | | | | | |
|---|---|---|---|---|---|---|---|---|---|---|---|---|---|
| | | Compound concentration mM | | | | | | | | | | | |
| | | 0 | 6.25 | 12.5 | 25 | 50 | 100 | 0 | 6.25 | 12.5 | 25 | 50 | 100 |
| First compound | L-ascorbic acid | × | 5 min. | 5 min. | 5 min. | 5 min. | 5 min. | 5 min. | 5 min. | 5 min. | 5 min. | 5 min. | 5 min. |
| | L-cysteine hydrochloride | | 5 min. | 5 min. | 5 min. | 5 min. | 5 min. | | 5 min. | 5 min. | 5 min. | 5 min. | 5 min. |
| | Glutathione | | 5 min. | 5 min. | 5 min. | 5 min. | 5 min. | | 5 min. | 5 min. | 5 min. | 5 min. | 5 min. |
| Second compound | DTT | | 8 min. | 8 min. | 8 min. | 8 min. | 8 min. | | 8 min. | 8 min. | 8 min. | 8 min. | 8 min. |
| | Thioglycolic acid | | 10 min. | 10 min. | 10 min. | 10 min. | × | | 8 min. | 8 min. | 8 min. | 10 min. | 15 min. |
| | Thioglycerol | | 5 min. | 5 min. | 5 min. | 5 min. | 5 min. | | 5 min. | 5 min. | 5 min. | 5 min. | 5 min. |
| | Mercaptoethanol | | 5 min. | 5 min. | 5 min. | 5 min. | 5 min. | | 5 min. | 5 min. | 5 min. | 5 min. | 5 min. |
| | TCEP | | 10 min. | 10 min. | 10 min. | 10 min. | × | | 8 min. | 10 min. | 10 min. | 10 min. | × |

| | | | | | | | | | | | | | |
|---|---|---|---|---|---|---|---|---|---|---|---|---|---|
| ×: 1 × 10⁴ cfu/mL undetectable | | | | | | | | | | | | | |

It has become clear that while sufficient sensitivity cannot be obtained in room temperature analysis for enzyme activity having an optimal temperature of 50 to 60°C, the present invention enables, by dry retention in a water absorbent material or addition to an sample of a first compound having an antioxidant effect or a compound having action of protecting SH groups and cleaving disulfide bonds, analyzing the enzyme activity in less than 10 minutes at a sensitivity equivalent to 50°C analysis even with room temperature analysis.

### (Example 3) Implementation of dry retention method with mixed addition of a first compound and a second compound

### (A) Mixed preparations of first compounds and second compounds

The first compounds and the second compounds were mixed in combinations shown in Table 3. Mixing concentrations were adjusted by dissolving 500 mM of each of a first compound and a second compound in 50 mM tris-maleate buffer solution pH 8.5 and thereafter mixing equivalent amounts of the first compound and the second compound such that the concentration of each would be 250 mM.

**[Table 3]**

| First compound | Second compound |
|---|---|
| L-ascorbic acid | DTT |
| L-ascorbic acid | Thioglycolic acid |
| L-ascorbic acid | Thioglycerol |
| L-ascorbic acid | Mercaptoethanol |
| L-ascorbic acid | TCEP |
| L-cysteine hydrochloride | DTT |
| L-cysteine hydrochloride | Thioglycolic acid |
| L-cysteine hydrochloride | Thioglycerol |
| L-cysteine hydrochloride | Mercaptoethanol |
| L-cysteine hydrochloride | TCEP |
| Glutathione | DTT |
| Glutathione | Thioglycolic acid |
| Glutathione | Thioglycerol |
| Glutathione | Mercaptoethanol |
| Glutathione | TCEP |

### (B) Dry retention of substrate and mixture of first compound and second compound

A solution with a first compound and a second compound mixed and the prepared substrate were mixed at a ratio of 1:9, and 10 mm paper disks (obtained from Advantec Co., Ltd.) were impregnated with 80 µL of the mixture per disk. The paper disks were then dried overnight at 25°C. (Final concentration of each of the first compound and the second compound mixed was 25 mM.)

### (C) Preparation of P. g bacteria

The bacterial culture solution after 24 hours of culturing was 10-fold serially diluted with 50 mM tris-maleate buffer solution pH 8.5 to prepare bacterial suspensions of 1.0 × 10⁶ cfu/mL, 1.0 × 10⁵ cfu/mL, 1.0 × 10⁴ cfu/mL, and 1.0 × 10³ cfu/mL.

### (D) Test method

Two of the paper disks, impregnated with the mixture of the first compound and the second compound and the substrate and then each impregnated with 80 µL of the prepared P. g bacterial suspension, were prepared and left to stand respectively for 2 minutes, 5 minutes, 8 minutes, 10 minutes, 15 minutes, and 20 minutes, one at room temperature and the other at 50°C. The former shall be referred to as room temperature analysis and the latter as 50°C analysis. Thereafter, 30 µL of the coloring solution was dripped onto each paper disk and coloration of each paper disk was observed and judged with the naked eye.

### (E) Results

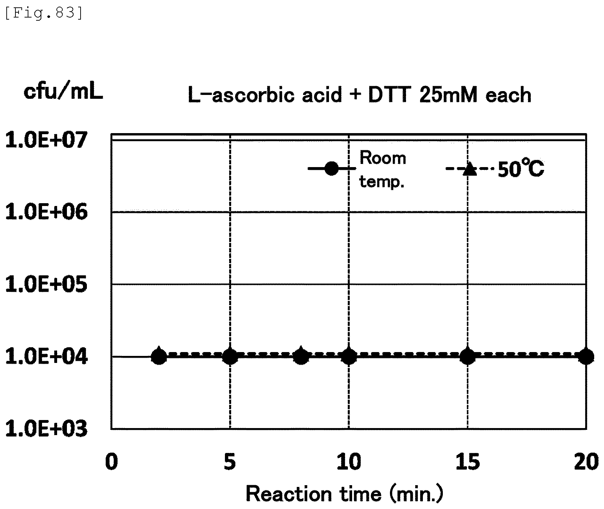

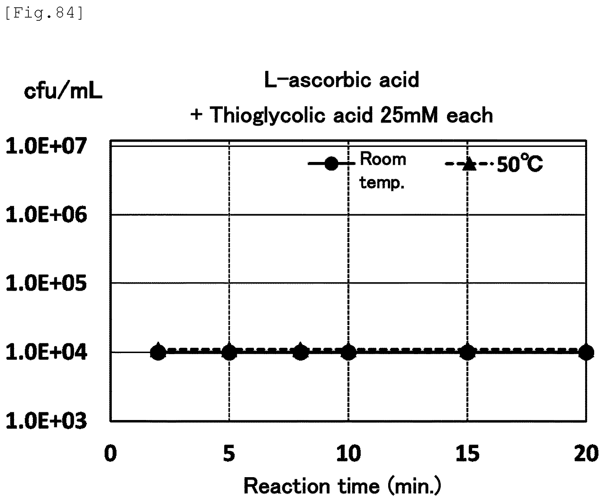

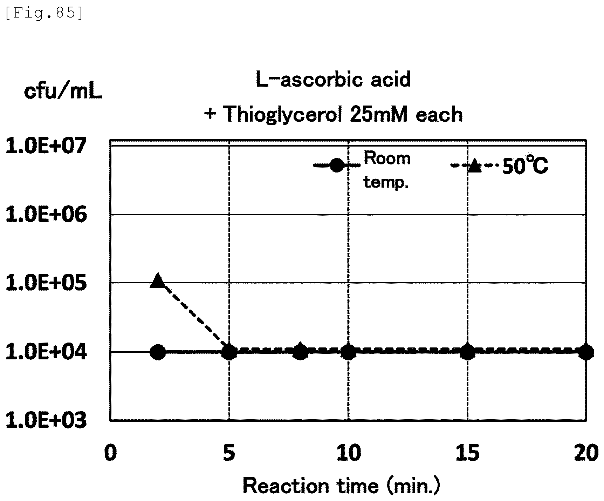

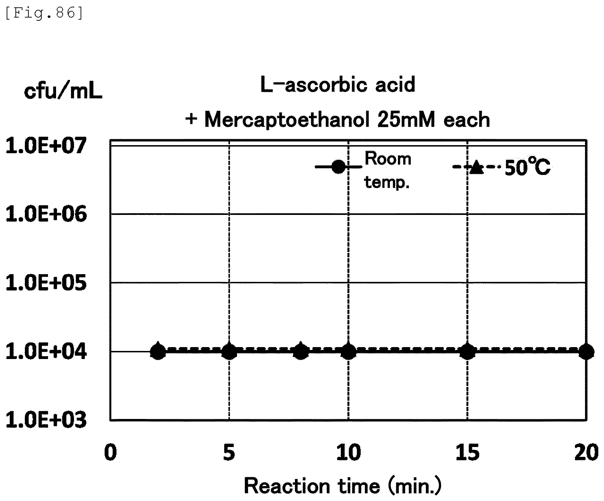

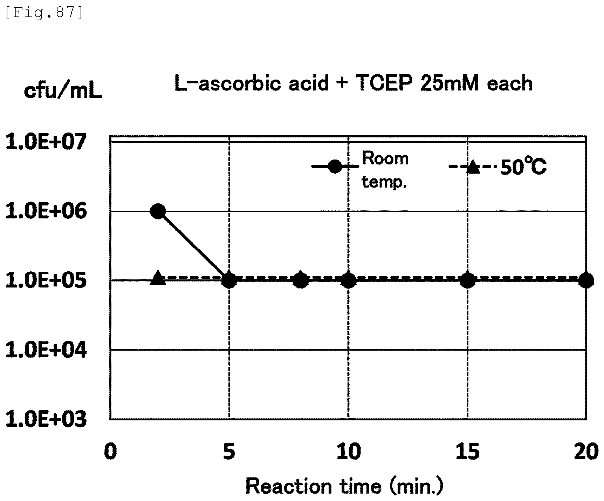

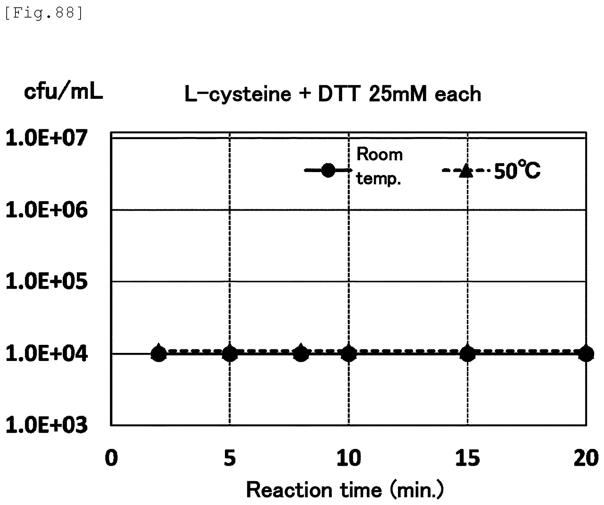

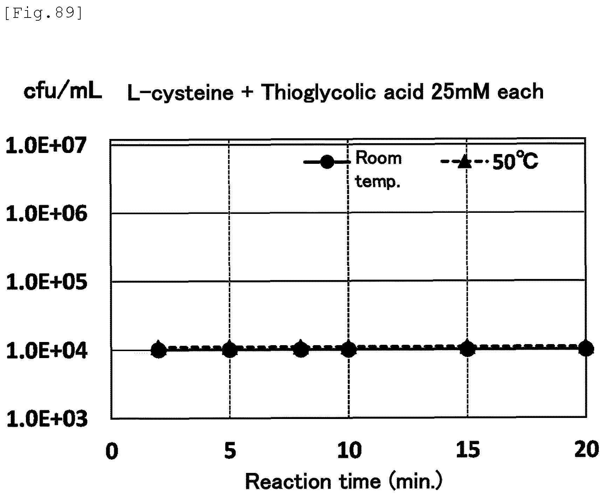

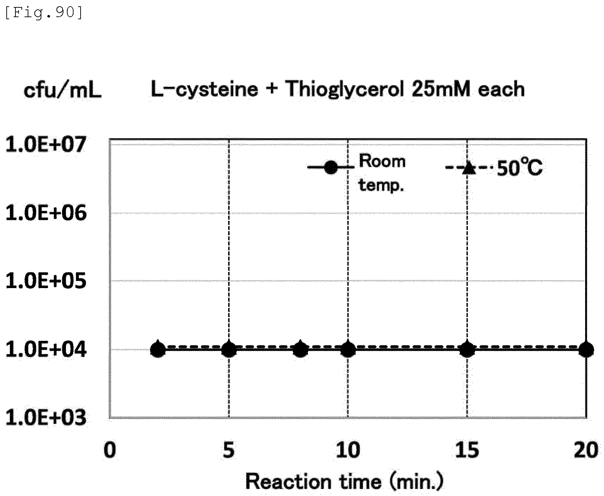

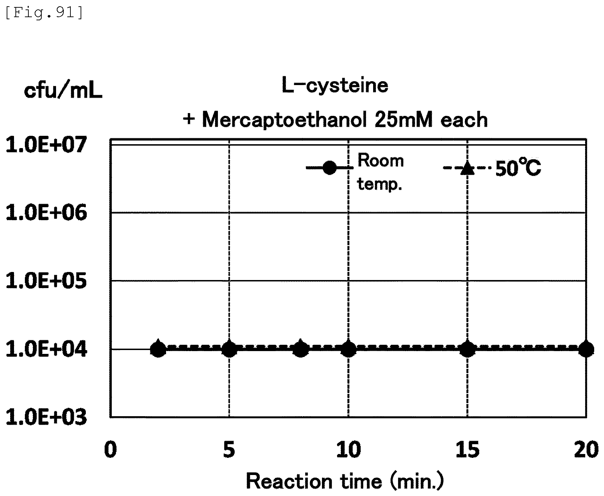

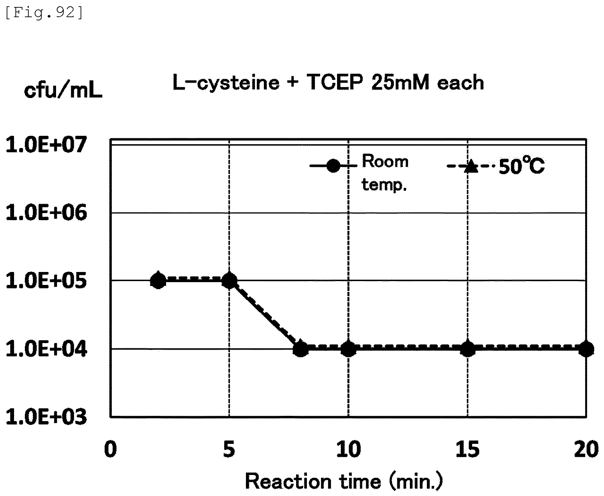

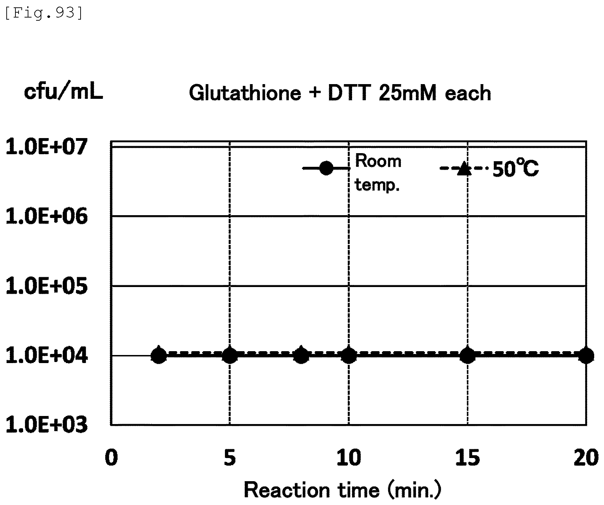

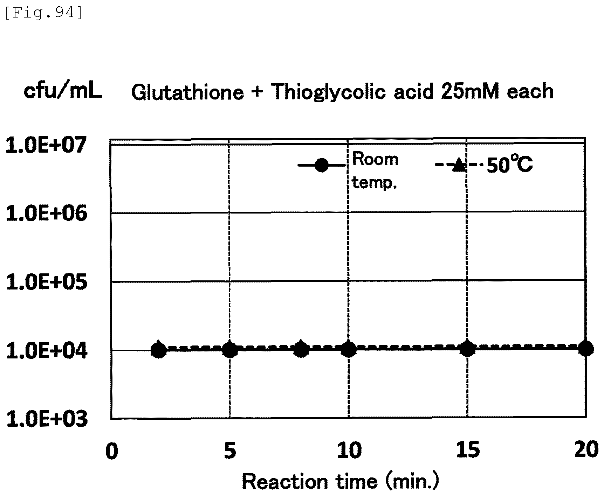

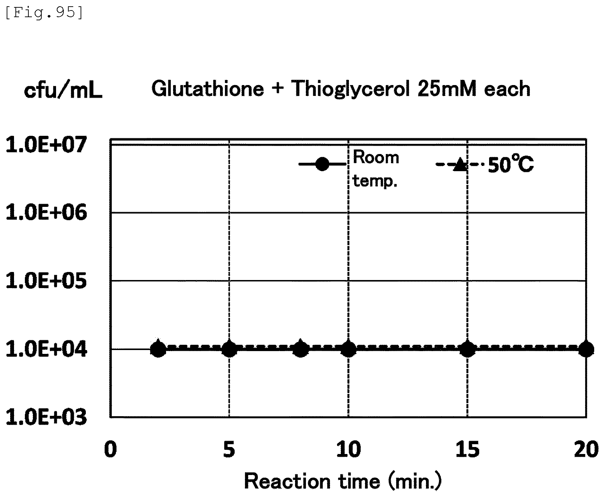

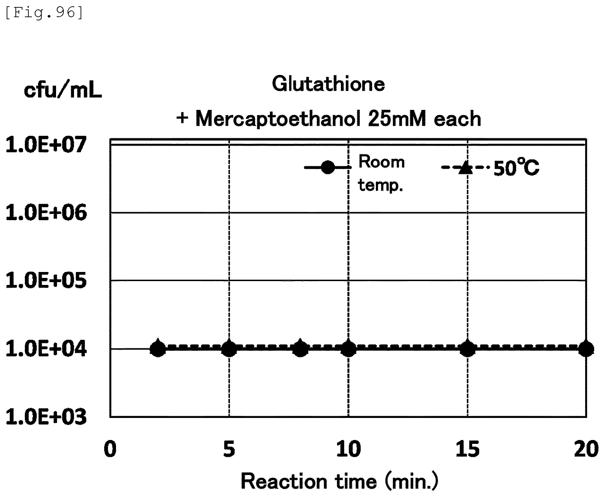

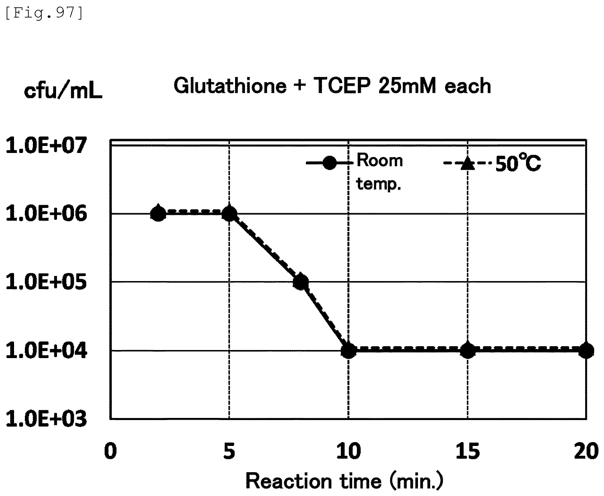

Fig. 83 to Fig. 97 show P.g detection sensitivities after adding mixtures of the first compound and second compound.

Table 4 shows minimum analysis times at which minimum bacterial concentrations could be detected with combinations of added mixtures of the first compound and second compound. As shown in the graphs and table, except for the combination of glutathione and TCEP, detection of the minimum bacterial concentration was possible in less than 10 minutes, similar to addition of the first compound or second compound alone. This indicated a result that detection is possible in less than 10 minutes by room temperature analysis and 50°C analysis at equivalent sensitivity even when the first compound and second compound are used in admixture, similar to their use alone.

**[Table 4]**

| Type of compound | | Room temperature analysis | | 50°C analysis | |
|---|---|---|---|---|---|
| | | First compound and second compound concentration mM | | | |
| First compound | Second compound | 0 | 25+25 | 0 | 25+25 |
| L-ascorbic acid | DTT | × | 2 min. | 5 min. | 2 min. |
| | Thioglycolic acid | | 2 min. | | 2 min. |
| | Thioglycerol | | 2 min. | | 5 min. |
| | Mercaptoethanol | | 2 min. | | 2 min. |
| | TCEP | | 5 min. | | 2 min. |
| L-cysteine hydrochloride | DTT | | 2 min. | | 2 min. |
| | Thioglycolic acid | | 2 min. | | 2 min. |
| | Thioglycerol | | 2 min. | | 2 min. |
| | Mercaptoethanol | | 2 min. | | 2 min. |
| | TCEP | | 8 min. | | 8 min. |
| Glutathione | DTT | | 2 min. | | 2 min. |
| | Thioglycolic acid | | 2 min. | | 2 min. |
| | Thioglycerol | | 2 min. | | 2 min. |
| | Mercaptoethanol | | 2 min. | | 2 min. |
| | TCEP | | 10 min. | | 10 min. |

| | | | | | |
|---|---|---|---|---|---|
| ×: 1 × 10⁴ cfu/mL undetectable | | | | | |

(Example 4) Implementation of solution method with mixed addition of a first compound and a second compound

### (A) Mixed preparations of first compounds and second compounds

The first compounds and the second compounds were mixed in combinations shown in Table 3. Mixing concentrations were adjusted by dissolving 55.5 mM of each of a first compound and a second compound in 50 mM tris-maleate buffer solution pH 8.5 and thereafter mixing equivalent amounts of the first compound and the second compound such that the concentration of each would be 27.75 mM.

### (B) Dry retention of substrate

The prepared substrate was mixed with 50 mM tris-maleate buffer solution pH 8.5 such that a volume ratio would be 9:1 and 10 mm paper disks (obtained from Advantec Co., Ltd.) were impregnated with 80 µL of the mixture per disk. The paper disks were then dried overnight at 25°C.

### (C) Preparation of P.g

The bacterial culture solution after 24 hours of culturing was 10-fold serially diluted with the prepared mixtures of first compounds and second compounds to prepare bacterial suspensions of 1.0 × 10⁶ cfu/mL, 1.0 × 10⁵ cfu/mL, 1.0 × 10⁴ cfu/mL, and 1.0 × 10³ cfu/mL.

### (D) Test method

Two of the paper disks, impregnated with the substrate and then each impregnated with 80 µL of the prepared P. g bacterial suspension, were prepared and left to stand respectively for 2 minutes, 5 minutes, 8 minutes, 10 minutes, 15 minutes, and 20 minutes, one at room temperature and the other at 50°C. The former shall be referred to as room temperature analysis and the latter as 50°C analysis. Thereafter, 30 µL of the coloring solution was dripped onto each paper disk and coloration of each paper disk was observed and judged with the naked eye.

### (E) Results

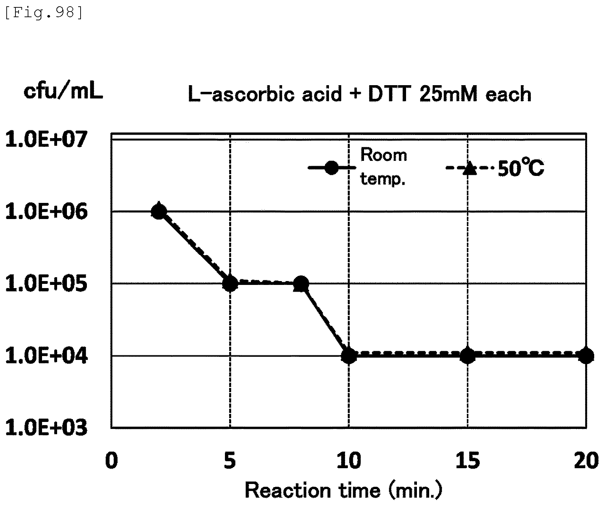

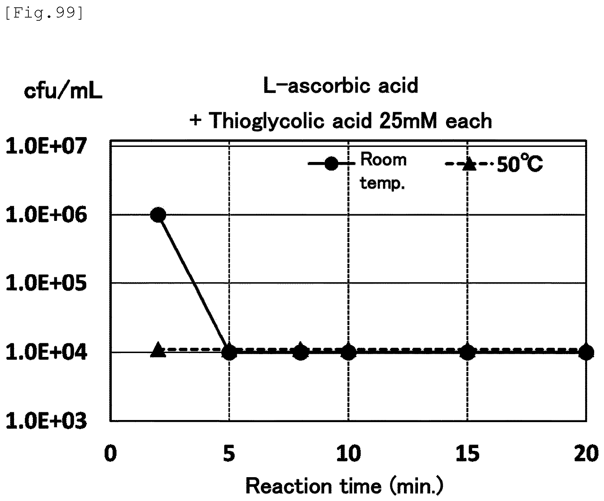

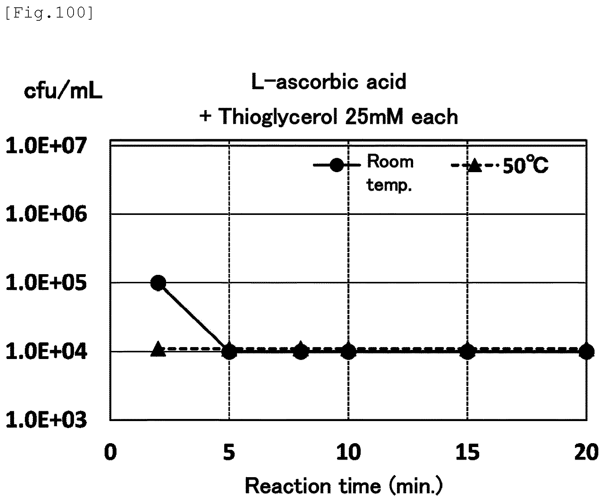

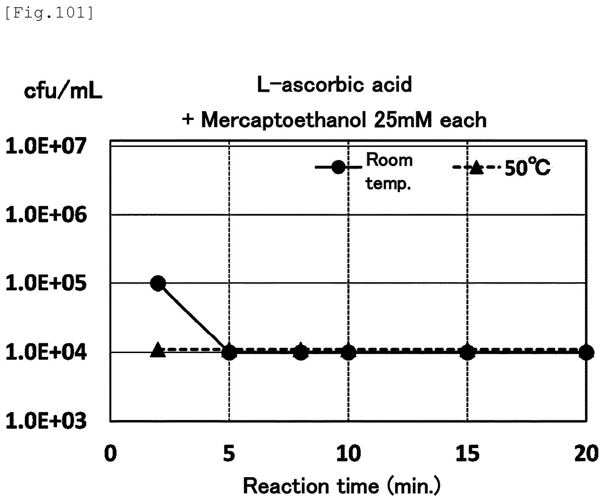

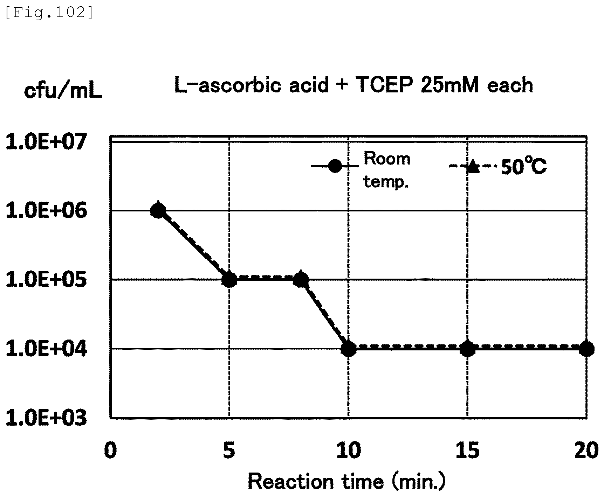

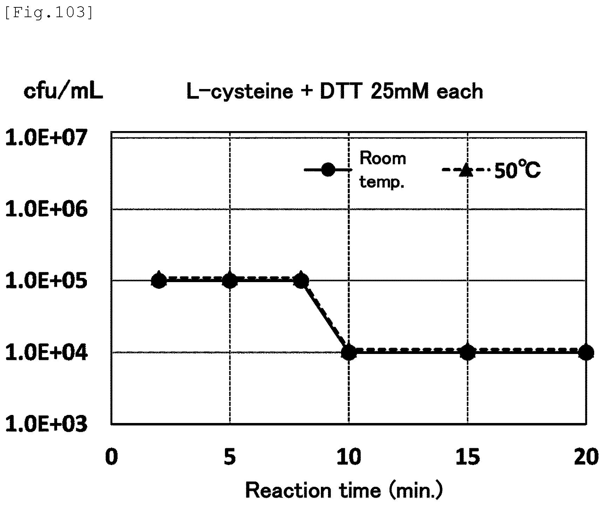

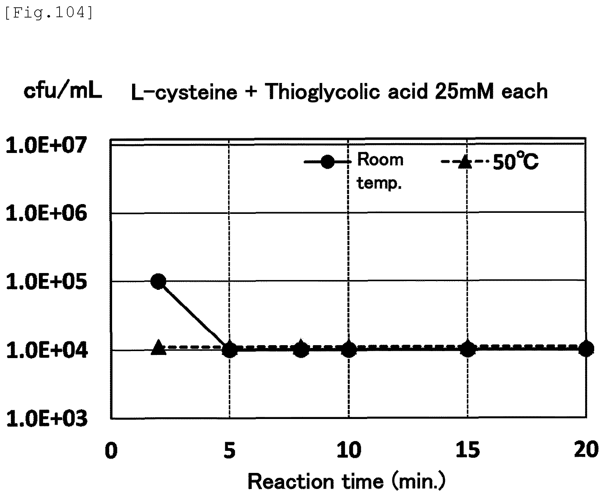

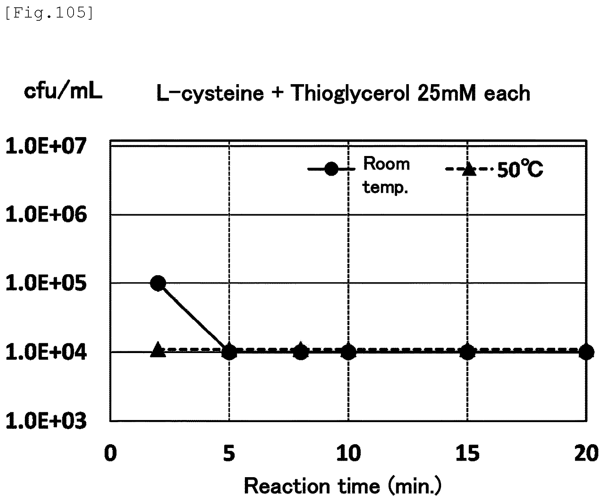

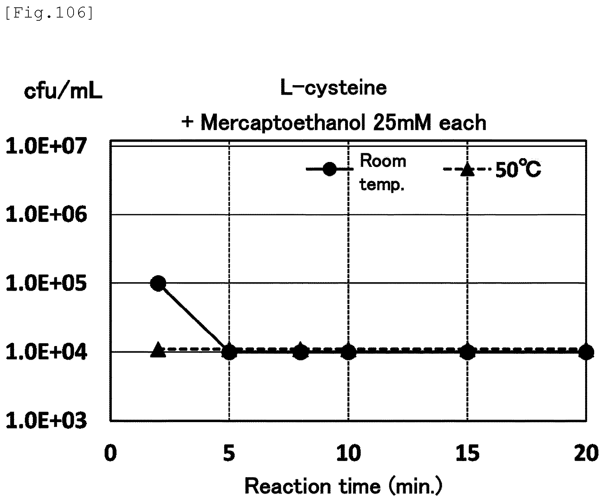

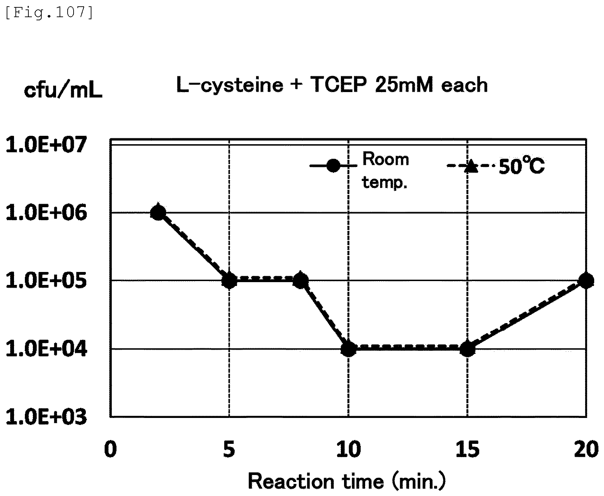

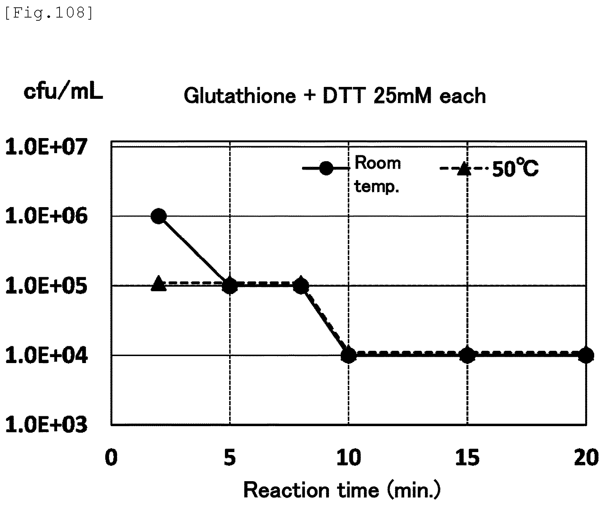

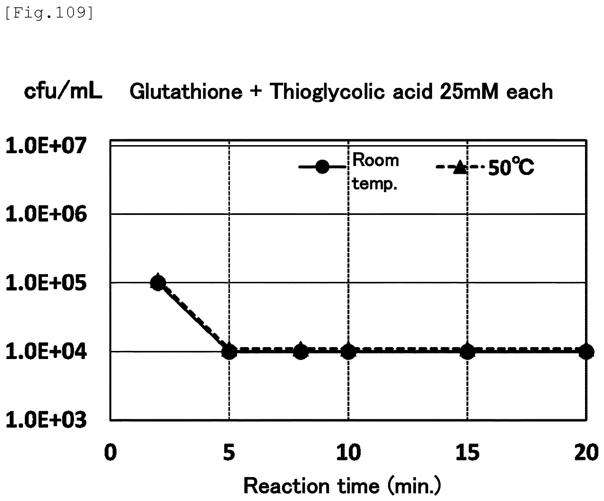

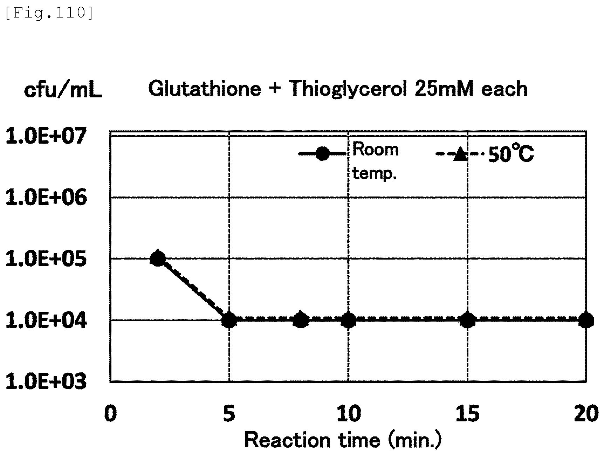

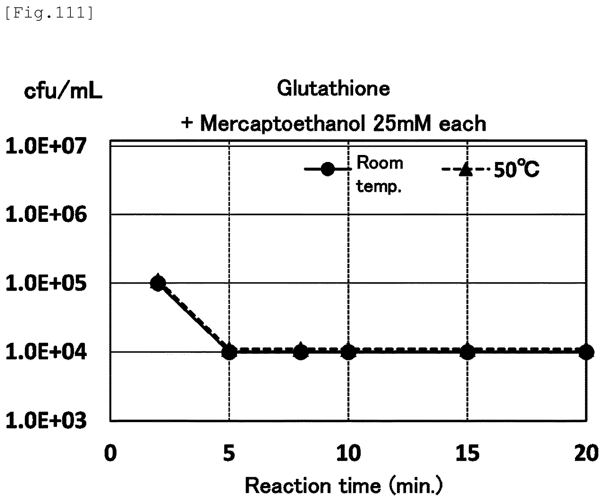

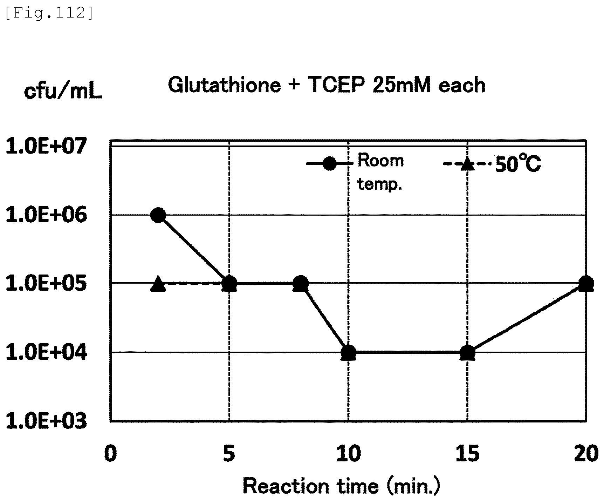

Fig. 98 to Fig. 112 show P.g detection sensitivities after adding mixtures of the first compound and second compound. Table 5 shows minimum analysis times at which minimum bacterial concentrations could be detected with combinations of added mixtures of the first compound and second compound. As shown in the graphs and table, except for the combinations of L-ascorbic acid/DTT, L-ascorbic acid/TCEP, L-cysteine hydrochloride/DTT, L-cysteine hydrochloride/TCEP, glutathione/DTT, and glutathione/TCEP, it was confirmed that sensitivity was equivalent with room temperature analysis and 50°C analysis and detection of minimum bacterial concentration was possible in less than 10 minutes, similar to addition of the first compound or second compound alone.

**[Table 5]**

| Type of compound | | Room temperature analysis | | 50°C analysis | |
|---|---|---|---|---|---|
| | | First compound and second compound concentration mM | | | |
| First compound | Second compound | 0 | 25+25 | 0 | 25+25 |
| L-ascorbic acid | DTT | × | 10 min. | 5 min. | 10 min. |
| L-ascorbic acid | Thioglycolic acid | | 5 min. | | 2 min. |
| L-ascorbic acid | Thioglycerol | | 5 min. | | 2 min. |
| L-ascorbic acid | Mercaptoethanol | | 5 min. | | 2 min. |
| L-ascorbic acid | TCEP | | 10 min. | | 10 min. |
| L-cysteine hydrochloride | DTT | | 10 min. | | 10 min. |
| L-cysteine hydrochloride | Thioglycolic acid | | 5 min. | | 2 min. |
| L-cysteine hydrochloride | Thioglycerol | | 5 min. | | 2 min. |
| L-cysteine hydrochloride | Mercaptoethanol | | 5 min. | | 2 min. |
| L-cysteine hydrochloride | TCEP | | 10 min. | | 10 min. |
| Glutathione | DTT | | 10 min. | | 10 min. |
| Glutathione | Thioglycolic acid | | 5 min. | | 5 min. |
| Glutathione | Thioglycerol | | 5 min. | | 5 min. |
| Glutathione | Mercaptoethanol | | 5 min. | | 5 min. |
| Glutathione | TCEP | | 10 min. | | 10 min. |

| | | | | | |
|---|---|---|---|---|---|
| ×: 1 × 10⁴ cfu/mL undetectable | | | | | |

It has become clear that while sufficient sensitivity cannot be obtained in room temperature analysis for enzyme activity having an optimal temperature of 50 to 60°C and the analysis time is also lengthened, the present invention enables, by dry retention in a water absorbent material or addition to an sample of a first compound having an antioxidant effect or a compound having action of protecting SH groups and cleaving disulfide bonds, analyzing the enzyme activity in less than 10 minutes at a sensitivity equivalent to 50°C analysis even with room temperature analysis.

### INDUSTRIAL APPLICABILITY

The present invention is useful for technologies in periodontopathic bacteria detection and diagnosis for easily performing ascertainment to make risk judgments and checks of degree of progress of periodontal disease as well as checks of treatment effects, etc.

## Claims

1. A method for detecting periodontopathic bacteria in which periodontopathic bacteria are detected in a sample at room temperature and in 2 minutes, whereby:
a first compound having an antioxidant effect and a second compound having action of protecting SH groups and cleaving disulfide bonds, is contacted with the sample together with N-α-benzoyl-DL-arginine-2-naphthylamide hydrochloride at room temperature and the mixture is allowed to stand at room temperature for 2 minutes, to allow detection of periodontopathic bacteria in subsequent coloration analysis with a coloring reagent, wherein:
the first compound is one selected from among L-ascorbic acid, L-cysteine hydrochloride, and glutathione, and
the second compound is one selected from among DTT, thioglycolic acid, thioglycerol and mercaptoethanol.

2. A method for detecting periodontopathic bacteria in which periodontopathic bacteria are detected in a sample at room temperature and in 2 minutes, whereby:
a sample containing a first compound having an antioxidant effect and a second compound having action of protecting SH groups and cleaving disulfide bonds, is added to N-α-benzoyl-DL-arginine-2-naphthylamide hydrochloride and the mixture is allowed to stand at room temperature for 2 minutes, to allow detection of periodontopathic bacteria in subsequent coloration analysis with a coloring reagent, wherein:
the first compound is one selected from among L-ascorbic acid, L-cysteine hydrochloride, and glutathione, and
the second compound is one selected from among DTT, thioglycolic acid, thioglycerol and mercaptoethanol.

3. A method for detecting periodontopathic bacteria in which periodontopathic bacteria are detected in a sample at room temperature and in 5 minutes, whereby:
a first compound having an antioxidant effect and a second compound having action of protecting SH groups and cleaving disulfide bonds are mixed and added to a water absorbent material together with N-α-benzoyl-DL-arginine-2-naphthylamide hydrochloride and the sample is contacted with the water absorbent material, and then allowed to stand at room temperature for 5 minutes, to allow detection of periodontopathic bacteria in subsequent coloration analysis with a coloring reagent, wherein:
the first compound is one selected from among L-ascorbic acid, L-cysteine hydrochloride, and glutathione, and
the second compound is one selected from among thioglycolic acid, thioglycerol and mercaptoethanol.

4. A method for detecting periodontopathic bacteria in which periodontopathic bacteria are detected in a sample at room temperature and in 5 minutes, whereby:
after a first compound having an antioxidant effect and a second compound having action of protecting SH groups and cleaving disulfide bonds are mixed, the mixture is added to the sample, and then N-α-benzoyl-DL-arginine-2-naphthylamide hydrochloride is added to the sample and the mixture is allowed to stand at room temperature for 5 minutes, to allow detection of periodontopathic bacteria in subsequent coloration analysis with a coloring reagent, wherein:
the first compound is one selected from among L-ascorbic acid, L-cysteine hydrochloride, and glutathione, and
the second compound is one selected from among thioglycolic acid, thioglycerol and mercaptoethanol.

5. A method for detecting periodontopathic bacteria in which periodontopathic bacteria are detected in a sample at room temperature and in 2 minutes, whereby:
a first compound having an antioxidant effect and/or a second compound having action of protecting SH groups and cleaving disulfide bonds, is contacted with the sample together with N-α-benzoyl-DL-arginine-2-naphthylamide hydrochloride at room temperature and the mixture is allowed to stand at room temperature for 2 minutes, to allow detection of periodontopathic bacteria in subsequent coloration analysis with a coloring reagent, wherein:
the first compound is glutathione, and
the second compound is one selected from among DTT and thioglycolic acid.

6. The method for detecting periodontopathic bacteria according to any one of claims 1 to 5, wherein the concentration of the first compound is 6.25 mM to 100 mM.

7. The method for detecting periodontopathic bacteria according to any one of claims 1 to 6, wherein the concentration of the second compound is 6.25 mM to 100 mM.

## Patentansprüche

1. Verfahren zum Nachweis von parodontopathischen Bakterien, bei dem parodontopathische Bakterien in einer Probe bei Raumtemperatur und in 2 Minuten nachgewiesen werden, wobei:
eine erste Verbindung, die eine antioxidative Wirkung besitzt, und eine zweite Verbindung, die SH-Gruppen schützend und Disulfidbindungen spaltend wirkt, zusammen mit N-α-Benzoyl-DL-arginin-2-naphthylamid-Hydrochlorid bei Raumtemperatur mit der Probe in Kontakt gebracht und das Gemisch 2 Minuten bei Raumtemperatur stehen gelassen wird, so dass ein Nachweis parodontopathischer Bakterien in einer anschließenden Färbeanalyse mit einem Färbereagens ermöglicht wird, wobei:
es sich bei der ersten Verbindung um eine unter L-Ascorbinsäure, L-Cystein-Hydrochlorid und Glutathion ausgewählte Verbindung und
bei der zweiten Verbindung um eine unter DTT, Thioglykolsäure, Thioglycerin und Mercaptoethanol ausgewählte Verbindung handelt.

2. Verfahren zum Nachweis von parodontopathischen Bakterien, bei dem parodontopathische Bakterien in einer Probe bei Raumtemperatur und in 2 Minuten nachgewiesen werden, wobei:
eine Probe, die eine erste Verbindung, die eine antioxidative Wirkung besitzt, und eine zweite Verbindung, die SH-Gruppen schützend und Disulfidbindungen spaltend wirkt, enthält, zu N-α-Benzoyl-DL-arginin-2-naphthylamid-Hydrochlorid gegeben und das Gemisch 2 Minuten bei Raumtemperatur stehen gelassen wird, so dass ein Nachweis parodontopathischer Bakterien in einer anschließenden Färbeanalyse mit einem Färbereagens ermöglicht wird, wobei:
es sich bei der ersten Verbindung um eine unter L-Ascorbinsäure, L-Cystein-Hydrochlorid und Glutathion ausgewählte Verbindung und
bei der zweiten Verbindung um eine unter DTT, Thioglykolsäure, Thioglycerin und Mercaptoethanol ausgewählte Verbindung handelt.

3. Verfahren zum Nachweis von parodontopathischen Bakterien, bei dem parodontopathische Bakterien in einer Probe bei Raumtemperatur und in 5 Minuten nachgewiesen werden, wobei:
eine erste Verbindung, die eine antioxidative Wirkung besitzt, und eine zweite Verbindung, die SH-Gruppen schützend und Disulfidbindungen spaltend wirkt, gemischt und zusammen mit N-α-Benzoyl-DL-arginin-2-naphthylamid-Hydrochlorid zu einem wasserabsorbierenden Material gegeben werden und die Probe mit dem wasserabsorbierenden Material in Kontakt gebracht und dann 5 Minuten bei Raumtemperatur stehen gelassen wird, so dass ein Nachweis parodontopathischer Bakterien in einer anschließenden Färbeanalyse mit einem Färbereagens ermöglicht wird, wobei:
es sich bei der ersten Verbindung um eine unter L-Ascorbinsäure, L-Cystein-Hydrochlorid und Glutathion ausgewählte Verbindung und
bei der zweiten Verbindung um eine unter Thioglykolsäure, Thioglycerin und Mercaptoethanol ausgewählte Verbindung handelt.

4. Verfahren zum Nachweis von parodontopathischen Bakterien, bei dem parodontopathische Bakterien in einer Probe bei Raumtemperatur und in 5 Minuten nachgewiesen werden, wobei:
nach dem Mischen einer ersten Verbindung, die eine antioxidative Wirkung besitzt, und einer zweiten Verbindung, die SH-Gruppen schützend und Disulfidbindungen spaltend wirkt, das Gemisch und dann N-α-Benzoyl-DL-arginin-2-naphthylamid-Hydrochlorid zu der Probe gegeben werden und das Gemisch 5 Minuten bei Raumtemperatur stehen gelassen wird, so dass ein Nachweis parodontopathischer Bakterien in einer anschließenden Färbeanalyse mit einem Färbereagens ermöglicht wird, wobei:
es sich bei der ersten Verbindung um eine unter L-Ascorbinsäure, L-Cystein-Hydrochlorid und Glutathion ausgewählte Verbindung und
bei der zweiten Verbindung um eine unter Thioglykolsäure, Thioglycerin und Mercaptoethanol ausgewählte Verbindung handelt.

5. Verfahren zum Nachweis von parodontopathischen Bakterien, bei dem parodontopathische Bakterien in einer Probe bei Raumtemperatur und in 2 Minuten nachgewiesen werden, wobei:
eine erste Verbindung, die eine antioxidative Wirkung besitzt, und/oder eine zweite Verbindung, die SH-Gruppen schützend und Disulfidbindungen spaltend wirkt, zusammen mit N-α-Benzoyl-DL-arginin-2-naphthylamid-Hydrochlorid bei Raumtemperatur mit der Probe in Kontakt gebracht und das Gemisch 2 Minuten bei Raumtemperatur stehen gelassen wird, so dass ein Nachweis parodontopathischer Bakterien in einer anschließenden Färbeanalyse mit einem Färbereagens ermöglicht wird, wobei:
es sich bei der ersten Verbindung um Glutathion und
bei der zweiten Verbindung um eine unter DTT und Thioglykolsäure ausgewählte Verbindung handelt.

6. Verfahren zum Nachweis von parodontopathischen Bakterien nach einem der Ansprüche 1 bis 5, wobei die Konzentration der ersten Verbindung 6,25 mM bis 100 mM beträgt.

7. Verfahren zum Nachweis von parodontopathischen Bakterien nach einem der Ansprüche 1 bis 6, wobei die Konzentration der zweiten Verbindung 6,25 mM bis 100 mM beträgt.

## Revendications

1. Procédé de détection de bactéries parodontopathiques dans lequel des bactéries parodontopathiques sont détectées dans un échantillon à température ambiante et en 2 minutes, moyennant quoi :
un premier composé ayant un effet antioxydant et un second composé ayant une action de protection de groupes SH et clivant des liaisons disulfure, sont mis en contact avec l'échantillon avec du chlorhydrate de N-α-benzoyl-DL-arginine-2-naphtylamide à température ambiante et le mélange est laissé reposer à température ambiante pendant 2 minutes, afin de permettre la détection de bactéries parodontopathiques lors d'une analyse par coloration ultérieure avec un réactif colorant, dans lequel :
le premier composé est choisi parmi l'acide L-ascorbique, le chlorhydrate de L-cystéine et le glutathion, et
le second composé est choisi parmi le DTT, l'acide thioglycolique, le thioglycérol et le mercaptoéthanol.

2. Procédé de détection de bactéries parodontopathiques dans lequel des bactéries parodontopathiques sont détectées dans un échantillon à température ambiante et en 2 minutes, moyennant quoi :
un échantillon contenant un premier composé ayant un effet antioxydant et un second composé ayant une action de protection de groupes SH et clivant des liaisons disulfure, est ajouté à du chlorhydrate de N-α-benzoyl-DL-arginine-2-naphtylamide et le mélange est laissé reposer à température ambiante pendant 2 minutes, afin de permettre la détection de bactéries parodontopathiques lors d'une analyse par coloration ultérieure avec un réactif colorant, dans lequel :
le premier composé est choisi parmi l'acide L-ascorbique, le chlorhydrate de L-cystéine et le glutathion, et
le second composé est choisi parmi le DTT, l'acide thioglycolique, le thioglycérol et le mercaptoéthanol.

3. Procédé de détection de bactéries parodontopathiques dans lequel des bactéries parodontopathiques sont détectées dans un échantillon à température ambiante et en 5 minutes, moyennant quoi :
un premier composé ayant un effet antioxydant et un second composé ayant une action de protection de groupes SH et clivant des liaisons disulfure sont mélangés et ajoutés à un matériau absorbant l'eau avec du chlorhydrate de N-α-benzoyl-DL-arginine-2-naphtylamide et l'échantillon est mis en contact avec le matériau absorbant l'eau, puis laissé reposer à température ambiante pendant 5 minutes, afin de permettre la détection de bactéries parodontopathiques lors d'une analyse par coloration ultérieure avec un réactif colorant, dans lequel :
le premier composé est choisi parmi l'acide L-ascorbique, le chlorhydrate de L-cystéine et le glutathion, et
le second composé est choisi parmi l'acide thioglycolique, le thioglycérol et le mercaptoéthanol.

4. Procédé de détection de bactéries parodontopathiques dans lequel des bactéries parodontopathiques sont détectées dans un échantillon à température ambiante et en 5 minutes, moyennant quoi :
après mélange d'un premier composé ayant un effet antioxydant et d'un second composé ayant une action de protection de groupes SH et clivant des liaisons disulfure, le mélange est ajouté à l'échantillon, puis du chlorhydrate de N-α-benzoyl-DL-arginine-2-naphtylamide est ajouté à l'échantillon et le mélange est laissé reposer à température ambiante pendant 5 minutes, afin de permettre la détection de bactéries parodontopathiques lors d'une analyse par coloration ultérieure avec un réactif colorant, dans lequel :
le premier composé est choisi parmi l'acide L-ascorbique, le chlorhydrate de L-cystéine et le glutathion, et
le second composé est choisi parmi l'acide thioglycolique, le thioglycérol et le mercaptoéthanol.

5. Procédé de détection de bactéries parodontopathiques dans lequel des bactéries parodontopathiques sont détectées dans un échantillon à température ambiante et en 2 minutes, moyennant quoi :
un premier composé ayant un effet antioxydant et/ou un second composé ayant une action de protection de groupes SH et clivant des liaisons disulfure, sont mis en contact avec l'échantillon avec du chlorhydrate de N-α-benzoyl-DL-arginine-2-naphtylamide à température ambiante et le mélange est laissé reposer à température ambiante pendant 2 minutes, afin de permettre la détection de bactéries parodontopathiques lors d'une analyse par coloration ultérieure avec un réactif colorant, dans lequel :
le premier composé est le glutathion, et
le second composé est choisi parmi le DTT et l'acide thioglycolique.

6. Procédé de détection de bactéries parodontopathiques selon l'une quelconque des revendications 1 à 5, dans lequel la concentration du premier composé est comprise entre 6,25 mM et 100 mM.

7. Procédé de détection de bactéries parodontopathiques selon l'une quelconque des revendications 1 à 6, dans lequel la concentration du second composé est comprise entre 6,25 mM et 100 mM.
